# EUROPEAN PATENT SPECIFICATION

(11) **EP 1 116 036 B1**
(45) Date of publication and mention of the grant of the patent: **11.08.2004**
(21) Application number: 99948273.0
(22) Date of filing: 17.09.1999
(51) Int. Cl.: G01N 33/58, H05B 33/10

(54) **WATER-SOLUBLE FLUORESCENT SEMICONDUCTOR NANOCRYSTALS**
WASSERLÖSLICHE FLUORESZIERENDE HALBLEITERNANOKRISTALLE
NANOCRISTAUX SEMICONDUCTEURS FLUORESCENTS HYDROSOLUBLES

(30) Priority: 18.09.1998 US 156863; 18.09.1998 US 100947 P; 24.09.1998 US 160454; 24.09.1998 US 160458; 17.09.1999 US 397436; 17.09.1999 US 397428; 17.09.1999 US 397432; 18.09.1998 US 101046
(43) Date of publication of application: 18.07.2001
(73) Proprietor: MASSACHUSETTS INSTITUTE OF TECHNOLOGY, Cambridge, MA 02142-1324 (US)
(72) Inventor: BAWENDI, Moungi, G., Boston, MA 02116 (US); MIKULEC, Frederick V., Apartment 2, Somerville, MA 02144 (US); LEE, Jin-Kyu, Kwanak-Gu Seoul 151-057 (KR)
(74) Representative: Mallalieu, Catherine Louise
(86) International application number: PCT/US1999/021375
(87) International publication number: WO 2000/017655

(56) References cited:
- US-A- 5 751 018
- CHAN W C W ET AL: "Quantum dot bioconjugates for ultrasensitive nonisotopic detection" SCIENCE, 25 SEPT. 1998, AMERICAN ASSOC. ADV. SCI, USA, vol. 281, no. 5385, pages 2016-2018, XP002125871 ISSN: 0036-8075
- LAWLESS D ET AL: "Bifunctional capping of CdS nanoparticles and bridging to TiO2" JOURNAL OF PHYSICAL CHEMISTRY, vol. 99, 1995, pages 10329-10335, XP000829587 AMERICAN CHEMICAL SOCIETY, US ISSN: 0022-3654 cited in the application
- DABBOUSI B O: "(CDSE)ZNS CORE-SHELL QUANTUM DOTS: SYNTHESIS AND CHARACTERIZATIONS OF A SIZE SERIES OF HIGHLY LUMINESCENT NANOCRYSTALLITES" JOURNAL OF PHYSICAL CHEMISTRY. B, MATERIALS, SURFACES, INTERFACES AND BIOPHYSICAL,US,WASHINGTON, DC, vol. 101, no. 46, page 9463-9475 XP002095418 ISSN: 1089-5647

## Description

### Field of the Invention

This invention relates to water-soluble nanocrystalline materials that emit energy over a narrow range of wavelengths. In particular, the invention relates to water-soluble semiconductor nanocrystals that emit light in the visible and infrared energy range.

### Background of the Invention

Semiconductor nanocrystals (also known as Quantum Dot™ particles) whose radii are smaller than the bulk exciton Bohr radius constitute a class of materials intermediate between molecular and bulk forms of matter. Quantum confinement of both the electron and hole in all three dimensions leads to an increase in the effective band gap of the material with decreasing crystallite size. Consequently, both the optical absorption and emission of semiconductor nanocrystals shift to the blue (higher energies) as the size of the nanocrystals gets smaller.

Bawendi and co-workers have described a method of preparing monodisperse semiconductor nanocrystals by pyrolysis of organometallic reagents injected into a hot coordinating solvent (Murray et al. (1993) *J*. *Am*. *Chem*. *Soc*., **115**:8706). This permits temporally discrete nucleation and results in the controlled growth of macroscopic quantities of nanocrystals. Size-selective precipitation of the crystallites from the growth solution can provide crystallites with even narrower size distributions. The narrow size distribution of the semiconductor nanocrystals allows the possibility of light emission with narrow spectral linewidths.

In an effort to improve the photoluminescent yield of the semiconductor nanocrystals, the nanocrystal surface has been passivated by reaction of the surface atoms of the nanocrystal with organic passivating ligands, to eliminate energy levels at the surface of the crystallite that lie within the energetically forbidden gap of the bulk interior. These surface energy states act as traps for electrons and holes which degrade the luminescence properties of the material. Such passivation produces an atomically abrupt increase in the chemical potential at the interface of the semiconductor and passivating layer *(see,* Alivisatos (1996) *J*. *Phys*. *Chem*. **100**:13226). Murray et al. (1993), *supra,* describes CdSe nanocrystals capped with organic moieties such as tri-*n*-octyl phosphine (TOP) and tri-*n*-octyl phosphine oxide (TOPO) with quantum yields as high as 20% in organic solvents such as toluene (*see, also*, doctoral thesis of Christopher Murray, "Synthesis and Characterization of II-VI Quantum Dots and Their Assembly into 3-D Quantum Dot Superlattices" (1995) Massachusetts Institute of Technology; and Kuno *et al*. (1997) *J*. *Phys*. *Chem*. **106**(23):9869).

Although semiconductor nanocrystals prepared as described by Bawendi and co-workers exhibit near monodispersity, and hence, high color selectivity, the luminescence properties of the material is process dependent. The stability of the photoluminescent property of the nanocrystal is a function of the nature of the passivating species coating the outer surface of the nanocrystal. Known organically coated nanocrystals are not robust and exhibit degradation of photoluminescent yield in solution. This is likely due to dissociation of the passivating layer from the surface of the nanocrystal or degradation of the passivating layer resulting in degradation of the semiconductor surface.

Passivation of semiconductor nanocrystals using inorganic materials also has been reported. Particles passivated with an inorganic coating are more robust than organically passivated particles and have greater tolerance to processing conditions necessary for their incorporation into devices. Previously reported inorganically passivated semiconductor nanocrystal particle structures include CdS-capped CdSe and CdSe-capped CdS (Than et al. (1996) *J*. *Phys*. *Chem*. **100**:8927); ZnS grown on CdS (Youn et al. (1988) *J*. *Phys*. *Chem*. **92**:6320); ZnS on CdSe and the inverse structure (Kortan et al. (1990) *J*. *Am*. *Chem*. *Soc.* **112**:1327); ZnS-capped CdSe nanocrystals (Hines et al. (1996) *J*. *Phys*. *Chem*. **100**:468; ZnSe-capped CdSe nanocrystals (Danek et al. (1996) *Chem*. *Materials* **8**:173); and SiO₂ on Si (Wilson et al. (1993) *Science* **262**:1242).

Kortan et al. (1990), *supra,* describes a ZnS capped-CdSe nanoparticle that has a layer of thiolphenyl groups bound to the outer surface. The thiolphenyl groups were used to passivate the surface and to allow the clusters to be isolated in powder form. Lawless et al. (1995) *J*. *Phys*. *Chem*. **99**: 1 0329 reported the preparation of CdS semiconductor nanocrystals capped with bifunctional mercaptocarboxylic acids HS(CH₂)ₙCOOH, wherein n is 1-3. TiO₂ particles were attached to the CdS nanocrystals through the functional carboxylic acid group of the bifunctional capping moiety in order to promote interparticle electron transfer between dissimilar semiconductor particles.

The semiconductor nanocrystals described above are soluble or dispersible only in organic solvents, such as hexane or pyridine. Many applications which rely on the fluorescent emission of the semiconductor nanocrystals require that the semiconductor nanocrystals be water-soluble.

Many reported water-soluble semiconductor nanocrystals suffer from significant disadvantages which limit their wide applicability. For example, Spanhel et al. (1987) *J. Am. Chem. Soc*. **109**:5649, discloses a Cd(OH)₂-capped CdS sol; however, the photoluminescent properties of the sol were pH dependent. The sol could be prepared on!y in a very narrow pH range (pH 8-10) and exhibited a narrow fluorescence band only at a pH of greater than 10. Such pH dependency greatly limits the usefulness of the material; in particular, it is not appropriate for use in biological systems.

Other groups have replaced the organic passivating layer of the semiconductor nanocrystal with water-soluble moieties; however, the resultant derivatized semiconductor nanocrystals are not highly luminescent. Short chain thiols such as 2-mercaptoethanol and 1-thio-glycerol have been used as stabilizers in the preparation of water-soluble CdTe nanocrystals. *See,* Rogach et al. (1996) *Ber. Bunsenges*. *Phys. Chem*. **100**:1772 and Rajh et al. (1993) *J. Phys. Chem.* **97**:11999. Other more exotic capping compounds have been reported with similar results. *See,* Coffer et al. (1992) *Nanotechnology* **3**:69 which describes the use of deoxyribonucleic acid (DNA) as a capping compound. In all of these systems, the coated semiconductor nanocrystals were not stable and photoluminescent properties degraded with time.

The unavailability of aqueous suspensions or solutions of semiconductor nanocrystals with sharp photoluminescent emissions limits their application in a variety of water-based applications, such as biological applications. In addition, aqueous solutions can often be very aggressive chemical systems and many of the known water-soluble semiconductor nanocrystal systems degrade, mainly by photoanodic decomposition at the semiconductor surface interface, during long exposure times in water.

Thus, there remains a need for water-soluble semiconductor nanocrystals that can be prepared as stable, robust suspensions or solutions in aqueous media. There is also a need for water-soluble semiconductor nanocrystals capable of energy emission with high quantum efficiencies, which possess a narrow particle size (and hence with narrow photoluminescence spectral range).

### Summary of the Invention

It is a primary aim of the invention to address the aforementioned needs in the art

It is another aim of the invention to provide water-soluble semiconductor nanocrystals that overcome the limitations of the prior art and that exhibit high quantum yields with photoluminescence emissions of high spectral purity

It is yet a further aim of the present invention to provide a semiconductor nanocrystal that is readily soluble in aqueous systems and that demonstrates chemical and electronic stability therein.

It is yet a further aim of the invention to provide a water-soluble semiconductor nanocrystal derivatized to provide linking or coupling capability.

In one aspect of the invention, a water-soluble semiconductor nanocrystal capable of energy emission is provided. According to the present invention there is provided a water-soluble semiconductor nanocrystal capable of energy emission, comprising:
a semiconductor nanocrystal core having a selected band gap energy;
a shell layer overcoating the semiconductor nanocrystal core, the shell comprised of a semiconductor material having a band gap energy greater than that of the core;
an outer layer comprising a molecule having a first portion comprising at least one linking group for attachment to the nanocrystal and a second portion comprising at least one hydrophilic group. The nanocrystal includes a semiconductor nanocrystal core having a selected band gap energy overcoated with a shell layer of a material having a band gap energy greater than that of the core and with appropriate band offsets. The water-soluble nanocrystal further comprises an outer layer at the outer surface of the overcoating layer. The outer layer includes a molecule having at least one linking group for attachment of the molecule to the overcoating layer and at least one hydrophilic group optionally spaced apart from the linking group by a hydrophobic region sufficient to minimize electron charge transfer across the hydrophobic region.

The outer layer of the nanocrystal can comprise an organic molecule. The organic molecule can be comprised of moieties selected to provide solubility in an aqueous medium, such as a long chain hydrocarbon terminating in a moiety having affinity for an aqueous medium, and a moiety that demonstrates an affinity to the semiconductor nanocrystal surface. The affinity for the nanocrystal surface promotes coordination of the organic molecule to the semiconductor nanocrystal outer surface and the moiety with affinity for the aqueous medium stabilizes the semiconductor nanocrystal suspension

In one preferred embodiment, the molecule has structural formula (I)

(I) H_{z}X¹((CH₂)ₙCO₂H)_{y}

and salts thereof, wherein. X¹ is N, P or O=P; n is greater than or equal to 6; and z and y are selected to satisfy the valence requirements of X¹.

In other preferred embodiments, the molecule has structural formula (II) 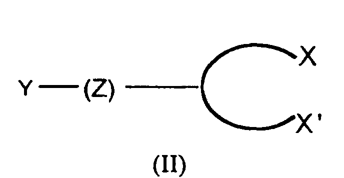 wherein: X and X' are the same or different and are selected from the group of S, N, P or O=P; Y is a hydrophilic moiety; and Z is absent or a hydrophobic region having a backbone of at least six atoms. X and X' can include other substituents to satisfy the valence requirements, such as for example, amines, thiols, phosphines and phosphine oxides, substituted by hydrogen or other organic moieties. In addition, the atoms bridging X and X' can be selected to form a 5-membered to 8-membered ring upon coordination to the semiconductor surface. The bridging atoms are typically carbon, but can be other elements, such as oxygen, nitrogen, and sulfur. Y can be any charged or polar group, such as a carboxylate, a sulfonates, a phosphate, a polyethylene glycol or other polyol and an ammonium salt, e.g., carboxylate (-CO₂⁻), sulfonate (SO₃⁻), hydroxide (-OH), alkoxides, ammonium salts (-NH₄⁺), and phosphate (-PO₄⁻²) and phosphonate (-PO₃⁻²), and the like. Z is typically an alkyl group or alkenyl group, but can also include other atoms, such as carbon and nitrogen. Z can be further modified as described herein to provide attractive interactions with neighboring ligands.

In yet another preferred embodiment, the molecule has structural formula (III): 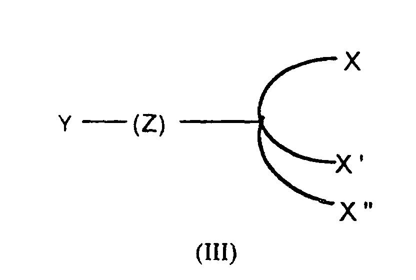 wherein X, X' and X" are the same or different and are selected from the group of S, N, P or O=P; Y is a hydrophilic moiety; and Z is a hydrophobic region having a backbone of at least six atoms. X, X' and X" can include other substituents in order to satisfy the valence requirements, such as for example, amines, thiols, phosphines and phosphine oxides, substituted by hydrogen or other organic moieties. In addition, the atoms bridging X, X' and X" can be selected to form a 5-membered to 8-membered ring upon coordination to the semiconductor surface. The bridging atoms are typically carbon, but can be other elements, such as oxygen, nitrogen, and sulfur. Y can be any charged or polar group, such as a carboxylate, a sulfonate, a phosphate, a polyethylene glycol or other polyol and an ammonium salt, e.g., carboxylate (-CO₂⁻), sulfonate (-SO₃⁻), hydroxide (-OH), alkoxides, ammonium salts (-NH₄⁺), phosphate (-PO₄⁻²), phosphonate (-PO₃⁻²), and the like. Z is typically an alkyl group or alkenyl group, but can also include other atoms, such as carbon and nitrogen. Z can be further modified as described herein to provide attractive interactions with neighboring ligands.

In other preferred embodiments, the molecule has structural formula (IV):

(IV) (R¹)ₐ-R²-[(R³)_{b}(R⁴)_{c}]_{d}

wherein:
R¹ is selected from the group consisting of heteroalkyl, heteroalkenyl, heteroalkynyl, -OR, -SR, -NHR, -NR'R", -N(O)HR, -N(O)R'R", -PHR, -PR'R", - P(NR'R")NR'R",-P(O)R'R", -P(O)(NR'R")NR'R", -P(O)(OR')OR", -P(O)OR, -P(O)NR'R", -P(S)(OR')OR", and -P(S)OR, wherein R, R' and R" are independently selected from the group consisting of H, a branched or unbranched alkyl, a branched or unbranched alkenyl, a branched or unbranched alkynyl, a branched or unbranched heteroalkyl, a branched or unbranched heteroalkenyl and a branched or unbranched heteroalkynyl, with the proviso that when *a* is greater than I the R¹ groups can be the same or different or can be linked to form a six-, seven-, eight-, nine- or ten-membered cycloalkyl, cycloalkenyl, heterocyclic, aryl, heteroaryl, or a six- to thirty-membered crown ether or heterocrown ether;
R² is selected from a bond (i.e., R² is absent), a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic, aryl and heteroaryl;
R³ is selected from a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic, aryl and heteroaryl;
R⁴ is selected from the group consisting of hydrogen, a carboxylate, a thiocarboxylate, an amide, an imide, a hydrazine, a sulfonate, a sulfoxide, a sulfone, a sulfite, a phosphate, a phosphonate, a phosphonium, an alcohol, a thiol, an amine, an ammonium, an alkyl ammonium, a nitrate, a sugar moiety, and a five-, six-, seven-, eight-, nine- or ten-membered cycloalkenyl, cycloalkynyl, heterocyclic, aryl, or heteroaryl;
*a* is 1, 2, 3 or 4;
*b* is 0, 1, 2 or 3;
*c* is 0, 1, 2 or 3; and
*d* is 0, 1, 2 or 3, wherein when *d* is 2 or 3 the R³ groups can be the same or different or can be linked together to form a five-, six-, seven-, eight-, nine- or ten-membered cycloalkyl, cycloalkenyl, heterocyclic, aryl, or heteroaryl.

Preferably, R¹ is a thiol (e.g., -SH), a phosphine, a phosphine oxide, or an amine (e.g., -NH2, -NHR or -NRR').

Preferably, R² contains between 6 and 20 atoms. More preferably, R² is a linear alkylene. alkenylene, alkynylene, heteroalkylene, heteroalkenylene or heteroalkynylene containing 6, 7, 8, 9, 10, 11, 12, 13, 14, 15, 16, 17, 18, 19 or 20 atoms, or a cycloalkyl or heterocyclic containing 5 or 6 atoms.

Preferably, when *b* is 1, 2 or 3, R³ contains between 6 and 20 atoms More preferably, R¹ is a linear alkylene, alkenylene, alkynylene, heteroalkylene, heteroalkenylene or heteroalkynylene containing 6, 7. 8, 9, 10, 11. 12, 13, 14, 15, 16, 17, 18, 19 or 20 atoms, or a cycloalkyl or heterocyclic containing 5 or 6 atoms.

Preferably, R⁴ is a carboxylate (-COO⁻), a phosphonate (-PO₃⁻). a sulfonate (-SO₃⁻) or an ammonium (-N⁺HRR').

In yet another embodiment of the invention, the molecule has structural formula (V):

(V) ⁅Y²(R¹)⁆ₘ.-R²⁅X²(R⁴)⁆_{n'}.

wherein pendant groups R¹ and R⁴ and the R² moiety are as defined above, X² and Y² are the same or different and are mer units selected from the group consisting of acrylate, styrene, imide, acrylamide, ethylene, vinyl, diacetylene, phenylene-vinylene, amino acid, sugar, sulfone, pyrrole, imidazole, thiophene and ether, and *m'* and *n'* are selected in relation to the number of available coordinating sites on the surface of the semiconductor nanocrystal. It is desirable that m' be no greater than the number of available coordinating sites and preferably no greater than about one-fourth of available coordinating sites. In particular, *m'* is in the range of about 3 to about 100. The value of *n'* is typically chosen to be commensurate with the value for *m'*. Thus, it is desirable that *n'* be no greater than the number of available coordinating sites and preferably no greater than about one-fourth of available coordinating sites. In particular, n' is in the range of about 3 to 100. The molecule can be a block copolymer, wherein a first block is provided that includes a pendant group capable of functioning as a linking moiety, Y. A second block is provided that includes a pendant group capable of functioning as a hydrophilic group, X. The polymer block serves as a hydrophilic region. In preferred embodiment, the molecule has the formula, 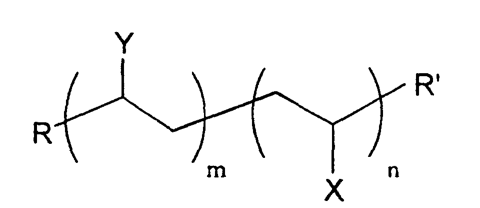 wherein the Xs are the same or different and are elements selected from the group of S, N, P or O=P; and the Ys are the same or different and are hydrophilic moieties, such as carboxylates, sulfonates, phosphates, phosphonates, polyethylene glycol, ammonium salt, and the like. X can include other substituents in order to satisfy the valence requirements, such as for example, amines, thiols, phosphine and phosphine oxides, substituted by hydrogen or other organic moieties. The terminal groups R and R' can be any moiety, including hydrogen. In particular, it is desirable for R to be a polar moiety due to its proximity to the hydrophilic block. Similarly, it is desirable for R' to be a non-polar moiety due to its proximity to the hydrophobic block. *m* and *n* are selected in relation to the number of available coordinating sites on the surface of the semiconductor nanocrystal. It is desirable that *m* be no greater than the number of available coordinating sites and preferably no greater than one-fourth of available coordinating sites. In typical applications, *m* is in the range of about 3 to 100. The value of n is typically chosen to be commensurate with the value for *m*. Thus, it is desirable that *n* be no greater than the number of available coordinating sites and preferably no greater than one-fourth of available coordinating sites. In typical applications, *n* is in the range of about 3 to 100.

Although not wishing to be bound by theory, the inventors believe that coordination of the molecule having structural formula (IV) to the overcoated nanocrystal occurs between surface moieties on the nanocrystal and the R¹ moiety of the molecule.

In another preferred embodiment, the water-solubilizing outer layer can comprise a homogeneous population of molecules having structural formula (I), (II), (III), (IV) or (V), a mixed population of molecules any individual structural formula, i.e., a mixed population of molecules all of which have structural formula (I), (II), (III), (IV) or (V), or a mixed population of molecules which have a combination of two or more of structural formulas (I), (II), (III), (IV) and (V)

In another aspect of the invention, a water-soluble semiconductor nanocrystal is provided in which the water solubilizing layer is a bilayer, having a first layer of the bilayer having affinity for the overcoating layer and a second layer of the bilayer having a hydrophobic region adjacent to the first layer and terminating in a hydrophilic group. The bilayer can include a coordinating lyophilic molecule used in the manufacture of the semiconductor nanocrystal as the first layer and a surfactant as the second layer.

These and other embodiments of the present invention will readily occur to those of ordinary skill in the art in view of the disclosure herein.

### Brief Description of the Drawing

The invention is described with reference to the figures, which are presented for the purpose of illustration only, and in which:
Figure 1 is a schematic illustration of the water-soluble nanocrystal of the invention;
Figure 2 is a schematic illustration of several alternative embodiments of the water-soluble layer of the nanocrystal;
Figure 3 is an illustration of a water-soluble nanocrystal of the invention having crosslinked hydrocarbon hydrophilic backbone;
Figure 4 is an illustration of a water-soluble nanocrystal of the invention comprising a polymethacrylate region;
Figure 5 is a schematic illustration of a bilayer water-soluble nanocrystal of the invention; and
Figure 6 is an illustration of the displacement reaction used in the formation of the water-soluble nanocrystal of the invention

### Detailed Description of the Invention

### Definitions and nomenclature.

Before the present invention is disclosed and described in detail, it is to be understood that this invention is not limited to specific assay formats, materials or reagents, as such may, of course, vary It is also to be understood that the terminology used herein is for the purpose of describing particular embodiments only and is not intended to be limiting.

It must be noted that, as used in the specification and the appended claims, the singular forms "a," "an" and "the" include plural referents unless the context clearly dictates otherwise. Thus, for example, reference to "a nanocrystal" includes more than one nanocrystal, reference to "an outer layer" includes more than one such outer layer, and the like.

In this specification and in the claims which follow, reference will be made to a number of terms which shall be defined to have the following meanings:

"Quantum dot™ particles" are a semiconductor nanocrystal with size-dependent optical and electronic properties. In particular, the band gap energy of a semiconductor nanocrystal varies with the diameter of the crystal.

"Semiconductor nanocrystal" includes, for example, inorganic crystallites between about 1 nm and about 1000 nm in diameter, preferably between about 2 nm and about 50 nm, more preferably about 5 nm to about 20 nm (such as about 5, 6, 7, 8, 9, 10, 11, 12, 13, 14, 15, 16, 17, 18, 19, or 20 nm) that includes a "core" of one or more first semiconductor materials, and which can be surrounded by a "shell" of a second semiconductor material. A semiconductor nanocrystal core surrounded by a semiconductor shell is referred to as a "core/shell" semiconductor nanocrystal. The surrounding "shell" material will preferably have a bandgap greater than the bandgap of the core material and can be chosen so to have an atomic spacing close to that of the "core" substrate. The core and/or the shell can be a semiconductor material including, but not limited to, those of the group II-VI (e.g., ZnS, ZnSe, ZnTe, CdS, CdSe, CdTe, HgS, HgSe, HgTe, MgTe and the like) and III-V (e.g., GaN, GaP, GaAs, GaSb, InN, InP, InAs, InSb, AlAs, AlP, AlSb, AlS, and the like) and IV (e.g., Ge, Si, Pb and the like) materials, and an alloy thereof, or a mixture, including ternary and quaternary mixtures, thereof.

A semiconductor nanocrystal is, optionally, surrounded by a "coat" of an organic capping agent The organic capping agent can be any number of materials, but has an affinity for the semiconductor nanocrystal surface. In general, the capping agent can be an isolated organic molecule, a polymer (or a monomer for a polymerization reaction). an inorganic complex, and an extended crystalline structure. The coat is used to convey solubility, e.g., the ability to disperse a coated semiconductor nanocrystal homogeneously into a chosen solvent, functionality, binding properties, or the like. In addition, the coat can be used to tailor the optical properties of the semiconductor nanocrystal.

"Quantum yield" as that term is used herein, means the ratio of photons emitted to that absorbed, e g., the photoluminescence quantum yield.

In other embodiments of the invention, the coated nanocrystal is characterized in that the nanocrystal exhibits less than a 10% rms (root mean square) and preferably less than 5% rms deviation in diameter of the core. Thus, the phrase "monodisperse particles" includes a population of particles wherein the population of particles deviate less than 10% rms in diameter and preferably less than 5% rms. The nanocrystal in an aqueous environment preferably exhibits photoluminescence having quantum yields of greater than 10%, and most preferably in the range of about 10% to 30%.

The term "alkyl" as used herein includes reference to a branched or unbranched saturated hydrocarbon group of 1 to 100 carbon atoms, such as methyl, ethyl, n-propyl, isopropyl, n-butyl, isobutyl, t-butyl, octyl, decyl, tetradecyl, hexadecyl, eicosyl, tetracosyl and the like, as well as cycloalkyl groups such as cyclopentyl, cyclohexyl and the like. The term "lower alkyl" includes an alkyl group of 1 to 20 carbon atoms, preferably 6 to 20 carbon atoms.

The term "alkylene" as used herein includes reference to a di-functional saturated branched or unbranched hydrocarbon chain containing from I to 100 carbon atoms, and includes, for example, methylene (-CH₂-), ethylene (-CH₂-CH₂-), propylene (-CH₂-CH₂-CH₂-), 2-methylpropylene (-CH₂-CH(CH₃)-CH₂-), hexylene (-(CH₂)₆-), and the like. "Lower alkylene" includes an alkylene group of 1 to 20, more preferably 6 to 20, carbon atoms.

The term "alkenyl" as used herein includes reference to a branched or unbranched hydrocarbon group of 2 to 100 carbon atoms containing at least one carbon-carbon double bond, such as ethenyl, n-propenyl, isopropenyl, n-butenyl, isobutenyl, t-butenyl, octenyl, decenyl, tetradecenyl, hexadecenyl, eicosenyl, tetracosenyl and the like. The term "lower alkenyl" includes an alkenyl group of 2 to 20 carbon atoms, preferably 6 to 20 carbon atoms, containing one -C=C- bond.

The term "alkenylene" includes reference to a difunctional branched or unbranched hydrocarbon chain containing from 2 to 100 carbon atoms and at least one carbon-carbon double bond "Lower alkenylene" includes an alkenylene group of 2 to 20, more preferably 6 to 20, carbon atoms, containing one carbon-carbon double bond.

The term "alkynyl" as used herein includes reference to a branched or unbranched hydrocarbon group of 2 to 100 carbon atoms containing at least one -C□C- bond, such as ethynyl, *n*-propynyl, isopropynyl, *n*-butynyl, isobutynyl, *t*-butynyl, octynyl, decynyl and the like. Preferred alkynyl groups herein contain 6 to 20 carbon atoms. The term "lower alkynyl" includes an alkynyl group of 2 to 10 carbon atoms, and one -C□C- bond.

The term "alkynylene" includes reference to a difunctional branched or unbranched hydrocarbon chain containing from 2 to 100 carbon atoms and at least one carbon-carbon triple bond. "Lower alkynylene" includes an alkynylene group of 2 to 10 carbon atoms, containing one -C□C- bond.

Optionally, an alkyl, alkylene, alkenyl, alkenylene, alkynyl or alkynyl chain can contain I to 6 linkages selected from the group consisting of -O-, -S- and -NR- wherein R is hydrogen, lower alkyl or lower alkenyl.

The terms "heteroalkyl," "heteroalkylene," "heteroalkenyl," "heteroalkenylene," "heteroalkynyl" and "heteroalkynylene" include reference to alkyl, alkylene, alkenyl, alkenylene, alkynyl and alkynylene groups, respectively, in which one or more of the carbon atoms have been replaced with, e.g., nitrogen, sulfur or oxygen atoms.

"Alkoxy" includes reference to the group -O-R, wherein R is an alkyl radical as defined above. Examples of an alkoxy radical include, but are not limited to, methoxy, ethoxy, isopropoxy and the like.

"Alkylamino" includes reference to a radical -NHR, wherein R is an alkyl radical as defined above Examples of alkylamino radicals include, but are not limited to, methylamino, (1-ethylethyl)amino, and the like

"Alkylthio" includes reference to a radical -SR where R is an alkyl radical as defined above. Examples of alkylthio radicals include, but are not limited to, methylthio, butylthio, and the like.

"Dialkylamino" includes reference to a radical -NR'R", wherein R' and R" are each independently alkyl radicals as defined above. Examples of dialkylamino radicals include, but are not limited to, dimethylamino, methylethylamino, diethylamino, di(1-methylethyl)amino, and the like.

"Hydroxyalkyl" includes reference to an alkyl radical as defined above, substituted with one or more hydroxy groups. Examples of hydroxyalkyl radicals include, but are not limited to, hydroxymethyl, 2-hydroxyethyl, 2-hydroxypropyl, 3-hydroxypropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl, and 2-(hydroxymethyl)-3-hydroxypropyl, and the like.

The term "acyl" as used herein includes reference to an alkyl group bound through a -(CO)- linkage. The term "lower acyl" includes an acyl group in which the alkyl group bound through the carbonyl linkage is a lower alkyl group.

The term "sugar moiety" includes reference to monosaccharides, disaccharides, polysaccharides, and the like. The term "sugar" includes those moieties which have been modified, e.g., wherein one or more of the hydroxyl groups are replaced with halogen, alkoxy moieties, aliphatic groups, or are functionalized as ethers, amines, or the like. Examples of modified sugars include: those which contain a lower alkoxy group in place of a hydroxyl moiety, i.e., α- or β-glycosides such as methyl α-D-glucopyranoside, methyl β-D-glucopyranoside, and the like; those which have been reacted with amines, i.e., N-glycosylamines or N-glycosides such as N-(α-D-glucopyranosyl)methylamine; those containing acylated hydroxyl groups, typically from I to 5 lower acyl groups; those containing one or more carboxylic acid groups, e.g., D-gluconic acid or the like; and those containing free amine groups such as D-glucosamine, D-galactosamine, N-acetyl-D-glucosamine or the like. Examples of preferred saccharides are glucose, galactose, fructose, ribose, mannose, arabinose, xylose. Examples of polysaccharides is dextran and cellulose

"Aryl" includes reference to a monovalent aromatic hydrocarbon radical consisting of one or more fused rings in which at least one ring is aromatic in nature, which can optionally be substituted with one or more of the following substituents: hydroxy, cyano, alkyl, alkoxy, thioalkyl, halo, haloalkyl, hydroxyalkyl, nitro, amino, alkylamino, and dialkylamino, unless otherwise indicated.

"Heteroaryl" includes reference to a monovalent aromatic carbocyclic radical having one or more rings incorporating one, two or three heteroatoms within the ring (chosen from nitrogen, oxygen, or sulfur) which can optionally be substituted with one or more of the following substituents: hydroxy, cyano, alkyl, alkoxy, thioalkyl, halo, haloalkyl, hydroxyalkyl, nitro, amino, and alkylamino and dialkylamino, unless otherwise indicated.

"Cycloalkyl" includes reference to a monovalent saturated carbocyclic radical consisting of one or more rings, which can optionally be substituted with one or more of the following substituents: hydroxy, cyano, alkyl, alkoxy, thioalkyl, halo, haloalkyl, hydroxyalkyl, nitro, amino, alkylamino and dialkylamino, unless otherwise indicated.

"Cycloalkenyl" includes reference to a monovalent unsaturated carbocyclic radical consisting of one or more rings and containing one or more carbon-carbon double bonds, which can optionally be substituted with one or more of the following substituents: hydroxy, cyano, alkyl, alkoxy, thioalkyl, halo, haloalkyl, hydroxyalkyl, nitro, amino, alkylamino and dialkylamino, unless otherwise indicated.

"Cycloalkynyl" includes reference to a monovalent unsaturated carbocyclic radical consisting of one or more rings and containing one or more carbon-carbon triple bonds, which can optionally be substituted with one or more of the following substituents: hydroxy, cyano, alkyl, alkoxy, thioalkyl, halo, haloalkyl, hydroxyalkyl, nitro, amino, alkylamino and dialkylamino, unless otherwise indicated.

"Heterocyclic" includes reference to a monovalent saturated carbocyclic radical, consisting of one or more rings, incorporating one, two or three heteroatoms (chosen from nitrogen, oxygen or sulfur), which can optionally be substituted with one or more of the following substituents: hydroxy, cyano, alkyl, alkoxy, thioalkyl, halo, haloalkyl, hydroxyalkyl, nitro, amino, alkylamino and dialkylamino, unless otherwise indicated.

The term "crown ether" includes reference to a saturated unbranched heterocyclic molecule, mono-, di-, tri-valent or higher (e.g., 4, 5, 6, 7, or 8) multivalent radical, Crown ethers are typically referred to as "x crown y" or "xCy" wherein x represents the total number of atoms in the molecule and y represents the number of heteroatoms in the molecule. Thus, for example, 12 crown 4 is a crown ether containing 12 atoms, 4 of which are heteroatoms and 18C6 is a crown ether containing 18 atoms, 6 of which are heteroatoms. Preferred heteroatoms are O, S and N, and in any particular crown ether the heteroatoms can be the same or different. A "heterocrown ether" is a crown ether in which the heteroatoms are different. Preferred crown ethers are six- to thirty-membered crown or heterocrown ethers, more preferred are 8C4, 9C3, 12C4, 15C5, 18C6 and 20C8, and even more preferred are 12C4 and 18C6.

"Optional" or "optionally" means that the subsequently described event or circumstance may or may not occur, and that the description includes instances where said event or circumstance occurs and instances where it does not. For example, the phrase "optionally substituted alkylene" means that an alkylene moiety may or may not be substituted and that the description includes both unsubstituted alkylene and alkylene where there is substitution, and the like.

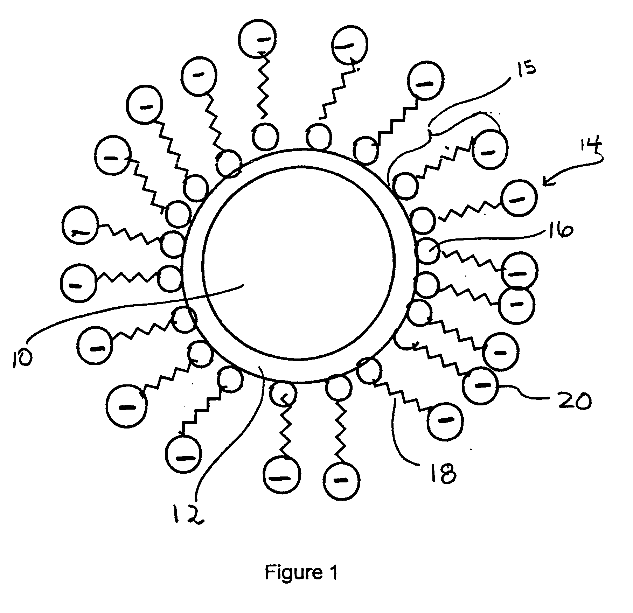

The present invention is directed to water-soluble semiconductor nanocrystals that are highly luminescent and stable in aqueous solutions. The nanocrystal is represented schematically in Figure 1. A semiconductor nanocrystal **10** is coated with an outer layer **14** that renders the crystal water-soluble. The outer layer **14** further is selected to maintain the luminescent properties of the nanocrystal and to improve the robustness of the nanocrystal in aqueous solutions. An optional overcoating layer **12** can be used to coat the semiconductor nanocrystal before application of the outer layer **14**. The outer layer includes a molecule **15** having at least one linking group **16** for attachment of the molecule to the overcoating layer and at least one hydrophilic group **20** spaced apart from the linking group by a hydrophobic region **18** sufficient to prevent electron charge transfer across the hydrophobic region. Note that the hydrophilic group **20** is denoted for the sake of convenience as a negative charge in Figure 1; however, the group can be positively charged or polar neutral.

The nanocrystal includes a semiconductor nanocrystal that demonstrates quantum confinement effects in their luminescent properties. These nanocrystals are known as "Quantum Dot™ particles". When semiconductor nanocrystals are illuminated with a primary energy source, a secondary emission of energy occurs of a frequency that corresponds to the band gap of the semiconductor material used in the semiconductor nanocrystal. In quantum confined particles, the band gap is a function of the size of the nanocrystal.

Upon exposure to a light source, the semiconductor nanocrystal emits energy of a wavelength characteristic of its composition and size. The water-soluble layer of the invention can be used with nanocrystals having various combinations of nanocrystal core and overcoating. The invention permits the preparation of a variety of water-soluble nanocrystals having a very narrow particle size distribution and exhibiting improvements in color purity and intensity of their photoluminescent emissions, as well as demonstrating robustness and stability in water-based suspensions and solutions. Most of the II-VI, III-V and group IV semiconductors have been prepared as quantum sized particles and exhibit quantum confinement effects in their physical properties and can be used in the water-soluble nanocrystals of the invention. Exemplary materials suitable for use as semiconductor nanocrystal cores include CdS, CdSe, CdTe, ZnS, ZnSe, ZnTe, MgTe, GaAs, GaP, GaSb, GaN, HgS, HgSe, HgTe, InAs, InP, InSb, InN, AlAs, AlP, AlSb, AlS, PbS, PbSe, Ge, Si, an alloy thereof, or a mixture thereof, including ternary and quaternary mixtures thereof.

The semiconductor nanocrystals are characterized by their uniform nanometer size. By "nanometer" size, it is meant less than about 150 Angstroms (Å), and preferably in the range of 15-150 Å. The nanocrystal also is substantially monodisperse within the broad size range given above. By monodisperse, as that term is used herein, it is meant a colloidal system in which the suspended particles have substantially identical size and shape. For the purposes of the present invention, monodisperse particles mean that at least 60% of the particles fall within a specified particle size range. In preferred embodiments, monodisperse particles deviate less than 10% rms in diameter, and preferably less than 5%. Monodisperse semiconductor nanocrystals have been described in detail in Murray et al. (1993), *supra*, the Murray thesis ( 1995), *supra*, and Kuno et al., *supra*.

In preferred embodiments, the semiconductor nanocrystal has an overcoating shell layer At the surface of the semiconductor nanocrystal, surface defects can result in traps for electron or holes that degrade the electrical and optical properties of the semiconductor nanocrystal. An insulating layer at the surface of the semiconductor nanocrystal provides an atomically abrupt jump in the chemical potential at the interface which eliminates energy states that can serve as traps for the electrons and holes This results in higher efficiency in the luminescent process.

Suitable materials for the overcoating shell layer include semiconductors having a higher band gap energy than the semiconductor nanocrystal. In addition to having a band gap energy greater than the semiconductor nanocrystals, suitable materials for the overcoating shell layer should have good conduction and valence band offset with respect to the semiconductor nanocrystal. Thus, the conduction band is desirably higher and the valance band is desirably lower than those of the semiconductor nanocrystal core. Thus, the core can be overcoated with a shell material comprising ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AIN, AlP, AlSb, an alloy thereof, or a mixture thereof, including ternary and quaternary mixtures thereof. Preferably, the band gap energy of the overcoating shell is greater than that of the core. For semiconductor nanocrystals that emit energy in the visible (e.g., CdS, CdSe, CdTe, ZnSe, ZnTe, GaP, GaAs) or near IR (e.g., InP, InAs, InSb, PbS, PbSe), a material that has a band gap energy in the ultraviolet regions can be used. Exemplary materials include ZnS, GaN, and magnesium chalcogenides, e.g., MgS, MgSe and MgTe. For semiconductor nanocrystals that emit in the near IR, materials having a band gap energy in the visible, such as CdS or CdSe, can also be used. The overcoating shell layer can include up to eight monolayers of the semiconductor material.

Particularly preferred semiconductor nanocrystals for emission in the visible include CdX³, wherein X³ is S, Se and Te and ZnY³, where Y³ is Se, Te. For those molecules, ZnS is a preferred material for use as the overcoating. For CdTe, ZnSe can be a preferred material for use as the overcoating due to the higher degree of lattice match between the materials. Overcoated nanocrystals which can be used in the present invention are described in Dabbousi et al. (1997)*J*. *Phys*. *Chem*. *B*, **101**(46)9463, and Kuno et al., *supra*.

Most prior art semiconductor nanocrystals are prepared in a coordinating solvent, resulting in the formation of a passivating organic layer on the nanocrystal surface comprised of the organic solvent. The passivated semiconductor nanocrystals thus are readily soluble in organic solvents, such as toluene, chloroform and hexane. The present invention provides a surface-modified particle that is soluble instead in aqueous media. According to the invention, the surface of the semiconductor nanocrystal is coated with an outer layer that stabilizes the semiconductor nanocrystal in aqueous solution. The outer layer includes a molecule having at least one linking moiety that attaches to the surface of the particle and that terminates in at least one hydrophilic moiety. The linking and hydrophilic moieties are optionally spaced apart by a hydrophobic region sufficient to prevent charge transfer across the region. The hydrophobic region also provides a "pseudo-hydrophobic" environment for the nanocrystal and thereby shields it from its aqueous surroundings. To exhibit high quantum efficiency it is desirable for the particles to remain electronically isolated from one another. The outer layer of the invention serves the additional useful purpose of maintaining the desired isolation between individual semiconductor nanocrystals.

The outer layer can be made up of any material that meets the structural and performance criteria stated herein. The material can be organic or inorganic. In preferred embodiments, the molecule is an organic molecule. In some embodiments, the outer layer can be a mixture of two or more different water-solubilizing molecules. In other embodiments, the outer layer can comprise additional molecules selected to provide a desirable attribute to the semiconductor nanocrystal. For example, the outer coating can include molecules having reactive functional groups for reaction with other substrates or molecules.

Suitable linking moieties include molecules having electron pairs available for interaction with the semiconductor surface, such as oxygen (O), sulfur (S), nitrogen (N) and phosphorus (P). Exemplary molecules include electron-donating moieties such as amines, thiols, phosphines, amine oxides, phosphine oxides, and the like. The linking moiety attaches to the semiconductor nanocrystal surface primarily through coordinate bonding of lone electron pairs of the nitrogen, sulfur. oxygen or phosphorous atom of the linking group. Covalent bonding and ionic bonding can also be used to form the interaction of the outer layer with the semiconductor surface.

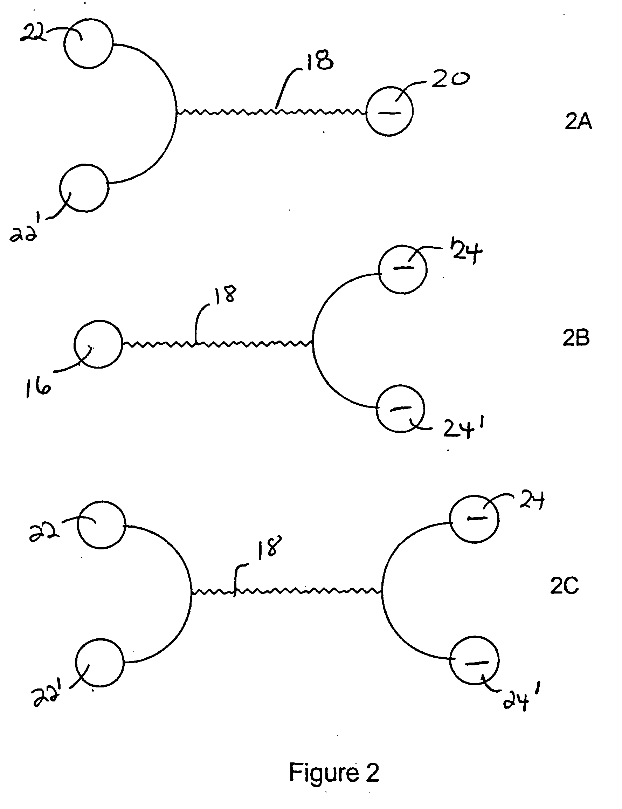

A molecule having a single linking moiety will result in the formation of an outer layer having water-solubilizing properties; however, it may be desirable for the molecule to comprise a plurality of linking moieties, as illustrated schematically in Figure 2A. Thus, the molecule can be a bidentate or tridentate ligand having two or more linking groups 22, 22'. Linking groups as described herein above can be used. For example, the molecule can be a derivatized dithiol, diamine, triamine, diphosphine, and the like. The linking groups can be the same or different.

Multidentate ligands provide enhanced stability and robustness to the organic layer and the resulting water-soluble nanocrystal. Without being bound to any particular mode of operation, it is believed that improved stability of the water-soluble nanocrystal is achieved by the increased binding coefficient of the multidentate ligand to the semiconductor surface. Since the organic layer is formed by an exchange reaction with solvated solvent molecules (see below), it follows that the water-solubilizing molecule can also be displaced from the surface of the semiconductor nanocrystal. It has been observed for example that the outer layer can be at least partially removed by dialysis of the water-soluble layer. Use of a multidentate ligand increases the strength of the interaction of the molecule with the semiconductor nanocrystal and decreases the ease of exchange of the organic layer with other coordinating molecules.

Increased stability of the resultant water-soluble semiconductor nanocrystal has been qualitatively observed in the size-selective precipitation of coated semiconductor nanocrystals. Semiconductor nanocrystals which have been overcoated with a bidentate ligand such as lipoic acid, exhibit a four-fold increase in suspension stability over a comparable monodentate ligand-coated molecule.

The hydrophilic moiety can be a polar or charged (positive or negative) group. The polarity or charge of the group provides the necessary hydrophilic interactions with water to provide stable solutions or suspensions of the semiconductor nanocrystal. Exemplary hydrophilic groups include polar groups such as hydroxides (-OH) , amines, polyethers, such as polyethylene glycol and the like, as well as charged groups, such as carboxylates (-CO₂⁻), sulfonates (-SO₃⁻), phosphates (-PO₄⁻²) and phosphonates(-PO₃⁻²), nitrates, ammonium salts (-NH₄⁺), and the like.

Water solubility has been achieved using molecules having a single hydrophilic group; however, it can be desirable for the molecule to include more than a single hydrophilic moiety, as illustrated schematically in Figure 2B. Figure 2B shows a molecule having at least two hydrophilic moieties **24, 24'**. The hydrophilic groups can be the same or different It is also contemplated that the water-solubilizing molecule can include multiple linking groups and hydrophilic groups, as shown in Figure 2C.

The hydrophobic region is selected to prevent photooxidation of the surface by charge transfer of a hole to the surface either from the core of the semiconductor nanocrystal or the environment. Typical processes include electrolysis of water from the environment with the resultant oxidation of sulfur or selenium (of the semiconductor nanocrystal) to SO₂ or SeO₂, , in instances where the semiconductor nanocrystal or overcoating layer contains S or Se. Transfer of a charge across the layer represents a non-energy emissive pathway for the excited state of the semiconductor and photoluminescence is thereby significantly reduced or quenched.

Prior art surface modifications of semiconductor nanocrystals include capping of CdS nanocrystals with 2-mercaptoethanol, 1-thioglycerol and 3-mercaptopropionic acid. See, Lawless et al., *supra*, and Rogach et al, *supra*. These short chain organic molecules do not provide a optimally luminescent, water-soluble semiconductor nanocrystal because the short carbon chain does not provide adequate insulation of the semiconductor nanocrystal against photooxidative processes. Therefore, charge transfer can occur between the semiconductor nanocrystal and either the carboxylate or the aqueous environment. Luminescence is partially quenched and quantum yields are low, i.e., less than 1%, in systems employing short chain organic molecules as a capping layer.

In one embodiment of the invention, the hydrophobic region is a long-chain hydrocarbon moiety, -(CH₂)ₙ-, where *n* is greater than six and preferably greater than eight. Hydrocarbon moieties wherein *n* is 11 or 15 have been successfully used in the manufacture of the water-soluble nanocrystal of the invention. There is no upper limit to the hydrocarbon chain length; however, it is recognized that very long hydrocarbon chains might render the nanocrystal undesirably "greasy". The hydrophobic region also can include branching hydrocarbons.

In another embodiment, the hydrophobic region can include a modified hydrocarbon backbone This modification can be the result of coupling reactions, e.g., carbodiimide coupling, used to increase the length of the hydrophobic backbone. Alternatively, non-carbon atoms can be introduced into the backbone to improve the attractive interaction of the water-solubilizing ligand with neighboring molecules.

The backbone also can be modified to include pendant groups that are attractive to neighboring hydrophobic regions through forces such as van der Waals attraction or hydrogen bonding. The attractive interaction between neighboring molecules serves to stabilize the outer layer of the semiconductor nanocrystal. In the event that the linking moiety should dissociate from the semiconductor surface, the attractive interaction with its neighbors will help the molecule to remain closely associated with the semiconductor nanocrystal until its linking moiety is able to recoordinate to the surface.

Exemplary modifications include amide, ketone, ether and aromatic moieties, and the like, substituting in whole or in part for the hydrocarbon backbone or attached as pendant groups from the hydrocarbon backbone. The polar nature of the moieties promotes hydrogen bonding and other attractive interaction with neighboring molecules which stabilizes the coating and increases its robustness in aqueous solution.

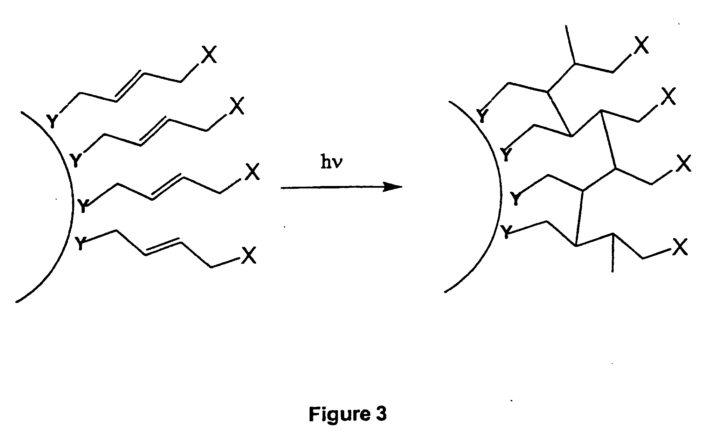

In other embodiments of the invention, the molecule of the outer layer is crosslinked to or polymerized with its neighboring molecules. Crosslinking provides stability to the layer by creating an effectively multidentate ligand across the semiconductor surface and significantly reducing ligand volatility and increasing the robustness and stability of the coating. Exemplary crosslinked networks are illustrated schematically in Figure 3.

To this end, the hydrocarbon chain can include some degree of unsaturation, which can be crosslinked upon exposure to uv energy or other free radical initiator to bridge neighboring ligands. Hydrocarbon unsaturation (and subsequent crosslinks) retain the hydrophobicity desired to prevent the photoinduced degradation of the semiconductor surface.

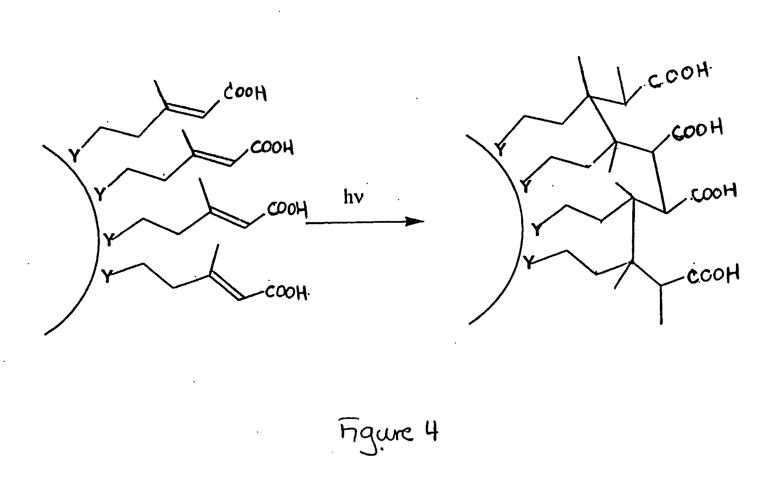

In one embodiment of the invention, the outer layer terminates in an unsaturated hydrophilic moiety that is capable of crosslinking or polymerizing. For example, the unsaturated moiety can be acrylic or methacrylate, which can be polymerized by exposure to free radical initiation, heat, UV energy, etc. to form poly(methacrylate), as is shown in Figure 4. The result is a polymer network, in this example, poly(methacrylate), that interacts with and effectively shields the semiconductor nanocrystal from an aqueous environment. The poly(methacrylate) can be deprotonated to provide a charged surface to render the nanocrystal water-soluble. Other exemplary unsaturated moieties for polymerization include acrylic acid and polystyrene derivatized to include a water-solubilizing functional group, e.g., carboxylate and sulfonate, and the like.

In another embodiment of the invention, the outer layer is comprised of a block copolymer that provides the requisite, linking, hydrophilic and hydrophobic functionalities. The copolymer includes at least a first block which contains a pendant group capable of functioning as a linking moiety and a second block having a pendant group capable of functioning as a hydrophilic moiety. The polymer backbone can function as the hydrophobic region. The linking and hydrophilic moieties can be directly attached to the hydrocarbon backbone or they can be attached through intermediary spacing groups. For example, the linking group Y can terminate from an aromatic or alkyl spacing group to provides greater access to the semiconductor surface.

In one embodiment of the invention, the molecule has structural formula (V):

(V) ⁅Y²(R¹)⁆_{m'}-R²⁅X²(R⁴)⁆_{n'}.

wherein R1, R2, R4, X², Y², *m'* and *n'* are as defined above. In one exemplary embodiment of a molecule having structural formula (V), the molecule is a block copolymer having the formula, 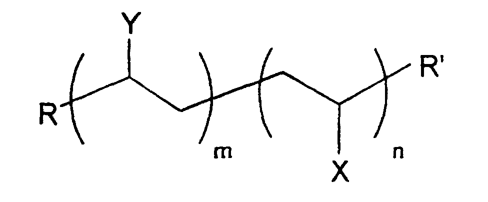 wherein X and Y are linking moieties and hydrophilic moieties, respectively, and can be any of the moieties discussed hereinabove. R and R¹ can be hydrogen, R can be a polar moiety and R' can be a non-polar moiety. The block copolymer can have a molecular weight of 300-50,000. The block sizes for the hydrophilic and linking moieties are preferably in the range of about 3 to 100.

Exemplary molecules for use in the invention have structural formula (I)

(I) H_{z}X((CH₂)ₙCO₂H)_{y}

wherein X, z, n and y are as defined above, structural formula (II) 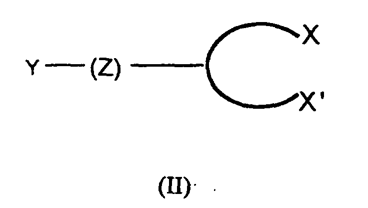 or structural formula (III) 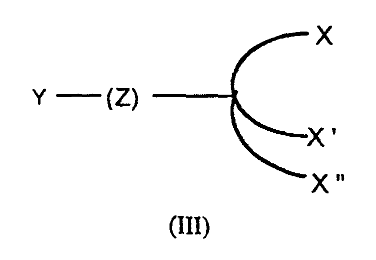 wherein Y, Z, X, X' and X" are as defined above, or structural formula (IV)

(IV) (R¹)ₐ-R²-[(R³)_{b}(R⁴)_{c}]_{d}

wherein R¹, R², R³, R⁴, *a*, *b*, *c*, and *d* are as defined above

Exemplary molecules for use in the outer layer of the water-soluble nanocrystal of the invention having the formula provided hereinabove include long chain aminocarboxylic acids, NH₂(CH₂)ₙCOOH, and phosphinocarboxylic acids, P((CH₂)ₙCOOH)_{3,} and their oxides O= P((CH₂)ₙCOOH)₃, wherein *n* is greater than or equal to 6, preferably n is greater than or equal to 8 and more preferably *n* is 10-12. The carboxylic acid can be deprotonated to provide the hydrophilic moiety. Other suitable molecules include bidentate ligands, such as, dihydrolipoic acid, HSCH₂CH₂CH(SH)(CH₂)₄COOH, or more generally, HSCH₂CH₂CH(SH)(CH₂)ₙCOOH, where *n* is 1-10 The length of the ligand can be increased by standard carbodiimide coupling methods, producing a species with the formula HSCH₂CH₂CH(SH)(CH₂)₄CONH(CH₂)ₙCOOH. The commercial availability of numerous precursors allows *n* to be easily varied from 2 to at least 10. Further detail of the carbodiimide coupling reaction can be found in Rich et al. (1979) The Peptides Vol. 1, Academic Press, pp. 241-2561.

Other suitable bidentate ligands include: the primary amine-containing analogues of the above molecule, H₂NCH₂CH₂CH(NH₂)(CH₂)ₙCOOH; derivatives of ethylene diamine, such as (HOOC(CH₂)ₙ)HNCH₂CH₂NH((CH₂)ₙCOOH); diphosphines such as (HOOC(CH₂)ₙ)₂PCH₂CH₂P((CH₂)ₙCOOH)₂; and the corresponding diphosphine oxides (HOOC(CH₂)ₙ)₂P(O)CH₂CH₂P(O)((CH₂)ₙCOOH)₂. An advantage to the use of the above-mentioned carboxylic acid derivatives it that they lend themselves to a wide range of chemistries. For example, the water-soluble semiconductor nanocrystal can be coupled with molecules having biological affinity for use in assaying. In another example, the water-soluble semiconductor nanocrystal can be coupled to beads, solid supports or objects of interest in order to track or identify an article. See US Patent Nos. 6,426,513 and 6,617,583, *supra*, for further details.

It will be readily apparent to one of ordinary skill in the art that the carboxylic acid moiety of the above-listed molecules can be substituted for a wide variety of charged or polar groups, including but not limited to, hydroxides, polyethers, such as polyethylene glycol and the like, and amines, as well as charged groups, such as carboxylates, sulfonates, phosphates, nitrates, ammonium salts and the like. Molecules such as listed herein above are commercially available or can be synthesized from methods and procedures well known in the art. It will be further apparent that the modifications described above with respect to hydrophobic regions and the hydrophilic groups can be incorporated into the molecule described immediately above in preparation of ligands suitable for use in the outer coating of the invention.

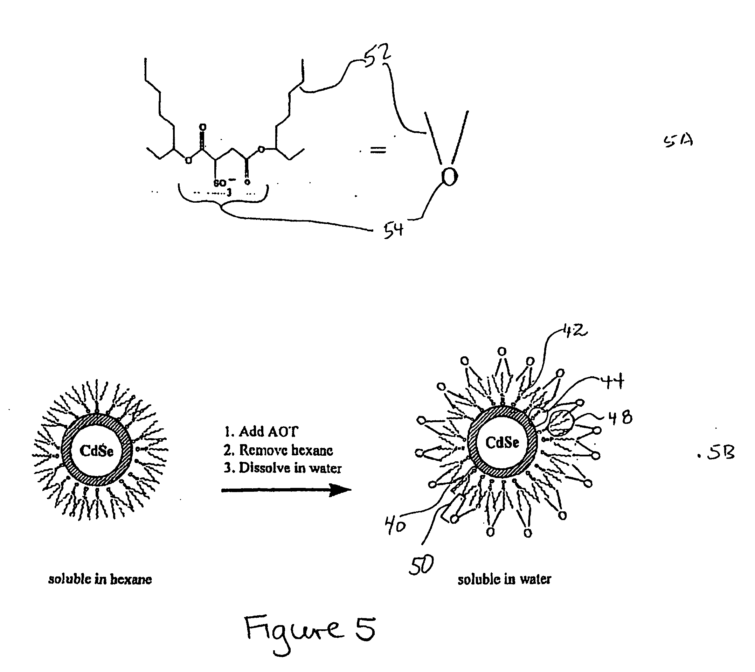

In another aspect of the invention, the water-soluble outer layer can be a bilayer comprising an inner layer having an affinity for the semiconductor surface and an outer layer terminating in a hydrophilic layer having an affinity for an aqueous medium. Figure 5A illustrates an exemplary molecule used in the outer bilayer of the invention. The molecule, dioctyl sulfosuccinate (aerosol OT ™), contains hydrophobic hydrocarbon regions **52** (denoted schematically as "-----" in Figure 5A) and a charged hydrophilic region **54** (denoted by "O" in Figure 5A). An exemplary bilayer molecule is shown in Figure 5B in which an inner layer **40** includes a molecule **42** (here TOPO) having a linking moiety **44** with an affinity for the semiconductor surface. A hydrophobic tail **48** extends from the linking moiety. The second outer layer **50** is comprised of a inner hydrophobic region **52** and an terminal hydrophilic moiety **54** for favorable interaction with an aqueous medium. The hydrophobic regions **48, 52** of the inner and outer layers, respectively, interact preferentially in the aqueous medium, to form a micelle encapsulating the nanocrystal therein. Figure 5B also illustrates the displacement reaction which occurs to form the bilayer of the invention.

The inner layer can include those coordinating solvents typically used in the manufacture of the semiconductor nanocrystal. Exemplary molecules include trialkyl phosphines and phosphine oxides, such as trioctylphosphine oxide (TOPO), trioctylphosphine (TOP), tributylphosphine (TBP), and the like. Hexadecylamine is a possible solvent, in particular, for solvating ZnSe.

The second outer layer can include any surfactant having a non-polar tail and a polar head. Non-limiting examples of surfactants include sodium dioctyl sulfosuccinate (known by the trade name AOT soap), C₁₂H₂₅(OCH₂CH₂)₂₃OH (Brij 35®), C₁₈H₃₇(OCH₂CH₂)₁₀OH (Brij 76®) and C₁₈H₃₇(OCH₂CH₂)₂₀ OH (Brij 98®). Even common hand soap, e g., lvory® soap, has been successfully used in the preparation of water-soluble nanocrystals of the invention.

A method for the preparation of the water-soluble nanocrystal follows. The method is described for a CdSe(ZnS), i.e., a CdSe core with a ZnS shell, semiconductor nanocrystal. but it is understood that the method can be applied in the preparation of semiconductor nanocrystals from the known semiconductor materials.

A population of nearly monodisperse nanocrystals first is prepared. The actual size of the nanocrystals will vary depending upon the material used. For CdSe, particles range in size from about 12 Å to about 150 Å diameter with a particle size distribution of about 5-10% rms in diameter. The monodisperse nanocrystals can be obtained using a high-temperature colloidal growth process, optionally followed by size-selective precipitation. If spectral emission linewidths are not as narrow as desired, size-selective precipitation can be used to obtain a population of semiconductor nanocrystals of narrower particle size distribution. *See*, Murray et al. (1993), *supra*, the Murray thesis (1995), *supra*, and Kuno et al., *supra*.

The semiconductor nanocrystal core can then be coated with the appropriate semiconductor overcoating layer, i.e., the shell. The coated nanocrystal can be prepared by introducing the substantially monodisperse first semiconductor nanocrystal and a precursor capable of thermal conversion into a second semiconductor material into a coordinating solvent. The coordinating solvent is maintained at a temperature sufficient to convert the precursor into the second semiconductor material yet insufficient to alter substantially the monodispersity of the first semiconductor manocrystal. Preferably, the second semiconductor material has a band gap greater than that of the first semiconductor nanocrystal. An overcoating shell of the second semiconductor material is formed on the first semiconductor nanocrystal. The monodispersity of the nanocrystal is monitored during conversion of the precursor and overcoating of the first semiconductor nanocrystal. The particle size distribution can be refined further by size-selective precipitation. Further details in the preparation of a coated semiconductor nanocrystal for use in the water-soluble nanocrystal of the invention can be found in US Patent No. 6,322,901, filed November 13, 1997 and entitled "Highly Luminescent Color-Selective Materials", and Dabbousi et al., *supra*.

The outer surface of the nanocrystal, as formed, includes an organic layer derived from the coordinating solvent used during the capping layer growth process. The nanocrystal surface can be modified to obtain the water-soluble nanocrystal of the invention by repeated exposure to an excess of a competing coordinating group For example, a dispersion of the semiconductor nanocrystal can be treated with a coordinating organic molecule, such as those described herein, to produce nanocrystals which disperse readily in water, but which no longer disperse in aliphatics. Such a surface exchange process can be carried out using a variety of molecules that are capable of coordinating or bonding to the outer surface of the capped semiconductor nanocrystal, such as by way of example, phosphines, thiols, amines, phosphine oxides and amine oxides.

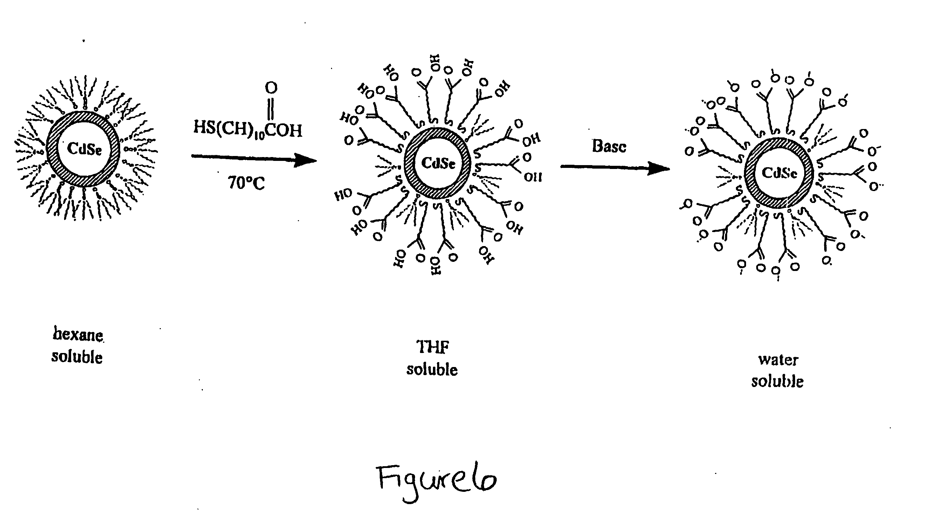

A typical reaction is illustrated in Figure 6. Semiconductor nanocrystals 60 are prepared in a coordinating organic solvent such as trioctylphosphine oxide (TOPO) which results in the formation of a passivating TOPO layer 62 on the surface of the semiconductor nanocrystal. This layer is displaced at least in part by the ligand 54, here represented as a long chain mercaptocarboxylic acid, comprising the outer layer of the invention in order to obtain water-soluble nanocrystal 66. Displacement can occur by dispersion of semiconductor nanocrystals or overcoated semiconductor nanocrystals in a medium containing high concentrations of the ligand used to form the outer coating. The medium can be a neat liquid comprising the ligand or it can be a highly concentrated solution. High concentrations drive the displacement reaction forward to maximize surface coverage of the nanocrystal by the molecule of the outer coating. Note that the displacement of the TOPO layer need not be complete in order to obtain a water-soluble nanocrystal.

Repeated exposure of the nanocrystal to the coordinating ligand solution may be desirable. The outer coating can be comprised of a mixture of the original polar organic solvent used in the preparation of the nanocrystal and the water-solubilizing molecule used in the outer coating of the invention. Substitution of the water-solubilizing molecule need only be sufficient to render the molecule water-soluble and need not be complete. In some embodiments, substitution is about 25-50% complete, preferably greater than 60% complete. The actual degree of substitution needed for solubility in water will depend on the number of charged or polar groups on the water-solubilizing molecule. Higher number of charged or polar groups can require a lower level of surface substitution in order to achieve water solubility.

It is also within the scope of the present invention to include other coordinating ligands on the outer coating of the nanocrystal. The additional ligands can be included to make available additional chemical reactions to the nanocrystal. For example coordinating ligands that terminate in reactive groups such as carboxylic acid. acyl halides and the like can be added to the outer surface of nanocrystal.

It is to be understood that while the invention has been described in conjunction with the preferred specific embodiments thereof, that the foregoing description as well as the examples which follow are intended to illustrate and not limit the scope of the invention. Other aspects, advantages and modifications within the scope of the invention will be apparent to those skilled in the art to which the invention pertains.

The following examples are intended to provide those of ordinary skill in the art with a complete disclosure and description of how to make and use the novel compositions of the invention, and are not intended to limit the scope of what the inventors regard as their invention in any way. Efforts have been made to ensure accuracy with respect to numbers used (e.g., amounts, temperatures, etc), but some experimental error and deviation should, of course, be allowed for. Unless indicated otherwise, parts are parts by weight, temperatures are in degrees centigrade, and pressure is at or near atmospheric.

The practice of the present invention will employ, unless otherwise indicated, conventional techniques of synthetic organic chemistry, biochemistry, molecular biology, and the like, which are within the skill of the art. Such techniques are explained fully in the literature. See, e.g., Kirk-Othmer's Encyclopedia of Chemical Technology; House's Modern Synthetic Reactions; the Marvel et al. text ORGANIC SYNTHESIS; Collective Volume 1, and the like.

### Example 1

### Preparation of TOPO-capped CdSe(ZnS)

(a) Preparation of CdSe. Trioctylphosphine oxide (TOPO, 90% pure) and trioctylphosphine (TOP, 95% pure) were obtained from Strem and Fluka, respectively. Dimethyl cadmium (CdMe₂) and diethyl zinc (ZnEt₂) were purchased from Alfa and Fluka, respectively, and both materials were filtered separately through a 0.2 m filter in an inert atmosphere box. Trioctylphosphine selenide was prepare by dissolving 0.1 mols of Se shot in 100ml of TOP thus producing a 1M solution of TOPSe. Hexamethyl(disilathiane) (TMS₂S) was used as purchased from Aldrich. HPLC grade n-hexane, methanol, pyridine and n-butanol were purchased from EM Sciences.

The typical preparation of TOP/TOPO-capped CdSe nanocrystals follows. TOPO (30g) was placed in a flask and dried under vacuum (∼1 Torr) at 180°C for 1 hour. The flask was then filled with nitrogen and heated to 350°C. In an inert atmosphere drybox the following injection solution was prepared: CdMe₂ (200 microliters, 2.78 mmol), 1 M TOPSe solution (4.0 mL, 4.0 mmol), and TOP (16 mL). The injection solution was thoroughly mixed, loaded into a syringe, and removed from the drybox.

The heat was removed from the reaction flask and the reagent mixture was delivered into the vigorously stirring TOPO with a single continuous injection. This produces a deep yellow/orange solution with a sharp absorption feature at 470-500 nm and a sudden temperature decrease to ∼240°C. Heating was restored to the reaction flask and the temperature was gradually raised to 260-280°C.

Aliquots of the reaction solution were removed at regular intervals (5-10 min) and absorption spectra taken to monitor the growth of the crystallites. The best samples were prepared over a period of a few hours steady growth by modulating the growth temperature in response to changes in the size distribution, as estimated from the sharpness of the features in the absorption spectra. The temperature was lowered 5-10°C in response to an increase in the size distribution. Alternatively, the reaction can also be stopped at this point. When growth appears to stop, the temperature is raised 5-10°C. When the desired absorption characteristics were observed, the reaction flask was allowed to cool to about 60°C and 20 mL of butanol were added to prevent solidification of the TOPO Addition of a large excess of methanol causes the particles to flocculate. The flocculate was separated from the supernatant liquid by centrifugation; the resulting powder can be dispersed in a variety of organic solvents (alkanes, ethers, chloroform, tetrahydrofuran, toluene, etc.) to produce an optically clear solution.

The powder can be further optimized in an optional size selective precipitation procedure. Nanocrystallites were dispersed in a solution of ∼10% butanol in hexane. Methanol was then added dropwise to this stirring solution until opalescence persisted. Separation of supernatant and flocculate by centrifugation produced a precipitate enriched with the largest crystallites in the sample. This procedure was repeated until no further sharpening of the optical absorption spectrum was noted. Size-selective precipitation can be carried out in a variety of solvent/nonsolvent pairs, including pyridine/hexane and chloroform/methanol.

(b) Preparation of CdSe(ZnS). A flask containing 5g of TOPO was heated to 190 C under vacuum for several hours then cooled to 60°C after which 0.5 mL trioctylphosphine (TOP) was added. Roughly 0.1-0.4 micromols of CdSe nanocrystals dispersed in hexane were transferred into the reaction vessel via syringe and the solvent was pumped off.

Diethyl zinc (ZnEt₂) and hexamethyldisilathiane ((TMS)₂S) were used as the Zn and S precursors, respectively. Particle size distribution for a particular sample was determined by comparison of the optical data to those of known semiconductor nanocrystals of known particle size. The amounts of Zn and S precursors needed to grow a ZnS shell of desired thickness for each CdSe sample was calculated based on the ratio of the shell volume to that of the core assuming a spherical core and shell and taking into account the bulk lattice parameters of CdSe and ZnS. For larger particles, the ratio of Zn to Cd necessary to achieve the same thickness shell is less than for the smaller nanocrystals. The actual amount of ZnS that grows onto the CdSe cores was generally less than the amount added due to incomplete reaction of the precursors and to loss of some material on the walls of the flask during the addition.

Equimolar amounts of the precursors were dissolved in 2-4 mL TOP inside an inert atmosphere glove box The precursor solution was loaded into a syringe and transferred to an addition funnel attached to the reaction flask. The reaction flask containing CdSe nanocrystals dispersed in TOPO and TOP was heated under an atmosphere of N₂. The temperature at which the precursors were added ranged from 140°C for 23Å diameter nanocrystals to 220°C for 55Å diameter nanocrystals. When the desired temperature was reached the Zn and S precursors were added dropwise to the vigorously stirring reaction mixture over a period of 5-10 minutes.

After the addition was complete the mixture was cooled to 90°C and left stirring for several hours. Butanol (5mL) was added to the mixture to prevent the TOPO from solidifying upon cooling to room temperature. The overcoated particles were stored in their growth solution to ensure that the surface of the nanocrystals remained passivated with TOPO They were later recovered in powder form by precipitating with methanol and redispersing into a variety of solvents including hexane, chloroform, toluene, THF and pyridine.

### Example 2

### Preparation of a water-soluble semiconductor nanocrystals using long chain mercaptocarboxylic acid.

TOPO-capped CdSe(ZnS) semiconductor nanocrystals were prepared as described in Example 1. The overcoated CdSe(ZnS) nanocrystals were precipitated from the growth solution using a mixture of butanol and methanol. To obtain the precipitated semiconductor nanocrystals, the solution was centrifuged for 5-10 min,, the supernatant was decanted and the residue was washed with methanol (2X).

The residue was weighed. The weight of the TOPO cap was assumed to be 30% of the total weight; and a 30-fold molar excess of the new capping molecule, 11-mercaptoundecanoic acid (MUA) was added The residue and MUA (neat solution) were stirred at 60°C for 8-12 hours. A volume of tetrahydrofuran (THF) equal to the added MUA was added to the MUA/nanocrystal mixture, while the mixture was still hot. A clear solution resulted and the coated semiconductor nanocrystals were stored under THF.

The coated semiconductor nanocrystals are rendered water-soluble by deprotonation of the carboxylic acid functional group of the MUA The deprotonation was accomplished by adding a suspension of potassium *t*-butoxide in THF to the MUA-semiconductor nanocrystal/THF solution. A gel resulted, which was then centrifuged and the supernatant liquid was poured off. The residue was washed twice with THF, centrifuged each time and the supernatant liquid poured off. The final residue was allowed to dry in air for 10 minutes. Deionized water (Millipore) was added to the residue until a clear solution formed.

The resultant coated semiconductor nanocrystals were tested for photoluminescent quantum yield. A CdSe semiconductor nanocrystal with a four-monolayer coating of ZnS coated as described had an absorption band a 480 nm and a photoluminescent band at 500 nm, with a quantum yield of 12%. A second CdSe semiconductor nanocrystal with a four monolayer coating of ZnS coated as described had an absorption band a 526 nm and a photoluminescent band at 542 nm, with a quantum yield of 18%.

### Example 3

### Preparation of a water-soluble semiconductor nanocrystal using a multidentate ligand.

A water-soluble semiconductor nanocrystal was prepared as described in Example 2, except that the bidentate ligand, dihydrolipoic acid was used.

The synthesis of a bidentate dithiol ligand was accomplished via the reduction of the coenzyme lipoic acid. The general procedure was described in Gunsalus et al. (1956) *J. Am. Chem. Soc*. **78**:1763-1766. Sodium borohydride (1.2 g) was added in 30-50 mg portions to a stirring suspension of lipoic acid (6.0 g) in 117 mL of 0.25 M sodium bicarbonate in 0°C water The reaction was stirred for 45 minutes at 0°C, after which 100 mL toluene was added and the mixture was acidified to pH ~ 2 with hydrochloric acid. The toluene layer was collected and saved. The aqueous layer was washed three times with 15 mL toluene. The organic layers were combined, dried with anhydrous magnesium sulfate, filtered, and the solvent removed under vacuum, leaving behind the product dihydrolipoic acid as a yellow oil (yield 80%).

Cap exchange was performed using the same procedure as described for 11-mercaptoundecanoic acid. TOPO-capped CdSe(ZnS) semiconductor nanocrystals were precipitated from solution and washed twice with methanol. The remaining powder was dissolved (under nitrogen) at 70°C in the minimum amount (usually 300-600 mg) of dihydrolipoic acid necessary to produce a clear solution. This mixture was stirred at 70°C for 6 hours, then stored at room temperature. The nanocrystals were rendered water soluble by treatment with potassium t-butoxide in THF, as described for the mercaptocarboxylic acid ligands.

### Example 4

### Preparation of a water-soluble semiconductor nanocrystal using a surfactant.

TOPO-capped CdSe(ZnS) semiconductor nanocrystals were prepared as described in Example 1. The semiconductor nanocrystals were dissolved in hexane to give a solution which was approximately 0.001-0.01 molar concentration of CdSe(ZnS) nanocrystals. Sufficient surfactant sodium dioctylsulfosuccinate (trade name AOT) was added to the mixture to produce a solution which is 5% surfactant by weight (but liquid Ivory® soap also worked). The hexane solvent was evaporated under vacuum. The resulting solid residue dissolved in water to give a clear solution whose quantum yield was approximately the same as the initial sample (∼75% of the original value).

## Claims

1. A water-soluble semiconductor nanocrystal capable of energy emission, compnsing:
a semiconductor nanocrystal core having a selected band gap energy;
a shell layer overcoating the semiconductor nanocrystal core, the shell comprised of a semiconductor material having a band gap energy greater than that of the core;
an outer layer comprising a molecule having a first portion comprising at least one linking group for attachment to the nanocrystal and a second portion comprising at least one hydrophilic group.

2. The water-soluble semiconductor nanocrystal of claim 1, wherein the outer layer further comprises
a bilayer overcoating the shell, the bilayer comprising:
an inner layer having affinity for the shell; and
an outer layer comprising a molecule having a hydrophilic group spaced apart from the inner layer by a hydrophobic region adjacent to the inner layer.

3. The water-soluble nanocrystal of claim 1, wherein the linking group comprises a moiety selected from the group consisting of amines, thiols, phosphines, phosphine oxides, and amine oxides

4. The water-soluble nanocrystal of claim 1, wherein the hydrophilic group is a charged or polar group

5. The water-soluble nanocrystal of claim 1, wherein the hydrophilic group is selected from the group consisting of carboxylic acid, carboxylate (-CO₂⁻), sulfonate (-SO₃), hydroxide (-OH), alkoxides, ammonium salts (-NH₄⁺), and phosphate (-PO₄⁻²) and phosphonate (-PO₃⁻²).

6. The water-soluble nanocrystal of claim 1, wherein the hydrophilic group comprises an unsaturated hydrophilic group that is crosslinkable or polymerizable.

7. The water-soluble nanocrystal of claim 6 ,wherein the unsaturated hydrophilic group is selected from the group consisting of methacrylic acid, acrylic acid, and hydrophilically derivatized styrene.

8. The water-soluble nanocrystal of claim 1, wherein the compound comprises two or more hydrophilic groups.

9. The water-soluble nanocrystal of claim 1, wherein the first portion is spaced apart from the second portion by a hydrophobic region.

10. The water-soluble nanocrystal of claim 9 ,wherein the hydrophobic region comprises a hydrocarbon chain of the formula -(CH₂)ₙ-, where n is greater than or equal to six.

11. The water-soluble nanocrystal of claim I or 2, wherein the molecule has structural formula (I),
(I) H_{z}X((CH₂)ₙCO₂H)_{y}
or a salt thereof, wherein
X is the first portion of the ligand and is S, N, P or O=P;
n is greater than or equal to 6; and
z and y are selected to satisfy the vaience requirements of X

12. The water-solub le nanocrystal of claim 1 or 2 ,wherein the molecule has structural formula (II), 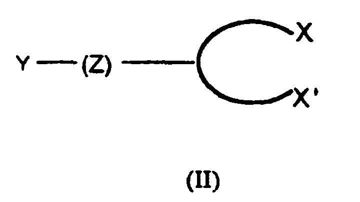 wherein:
Y is the hydrophilic moiety;
Z is a hydrophobic region having a backbone of at least six atoms;
X and X' are individually or together the linking groups, are the same or different and are selected from the group of S, N, P and O=P, or are linked together to form a 5-membered to 8-membered ring upon coordination to the nanocrystal surface.

13. The water-soluble nanocrystal of claim 1 or 2 ,wherein the molecule has structural formula (III), 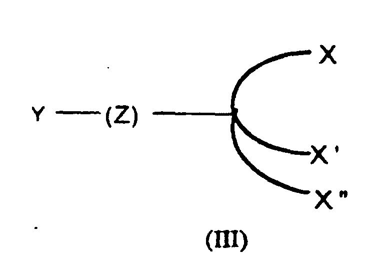 wherein
Y is the hydrophilic moiety;
Z is a hydrophobic region having a backbone of at least six atoms;
X, X' and X" are individually or together linking groups, are the same or different and are selected from the group of S, N, P and O=P, or are linked together to form a 5-membered to 8-membered ring upon coordination to the nanocrystal surface.

14. The water-soluble nanocrystal of claim 1, or 2 wherein the molecule has the structural formula (IV),
(IV) (R¹)ₐ-R²-[(R³)_{b}(R⁴)_{c}]_{d}
wherein:
R¹ is the first portion and is selected from the group consisting of heteroalkyl, heteroalkenyl, heteroalkynyl, -OR, -SR, -NHR, -NR'R", -N(O)HR, -N(O)R'R", -PHR, -PR'R", -P(NR'R")NR'R"-P(O)R'R", -P(O)(NR'R")NR'R", -P(O)(OR')OR", - P(O)OR, -P(O)NR'R", -P(S)(OR')OR", and -P(S)OR, wherein R, R' and R" are independently selected from the group consisting of H, a branched or unbranched alkyl, a branched or unbranched alkenyl, a branched or unbranched alkynyl, a branched or unbranched heteroalkyl, a branched or unbranched heteroalkenyl and a branched or unbranched heteroalkynyl, with the proviso that when *a* is greater than 1 the R¹ groups can be the same of different or can be linked to form a six-, seven-, eight-, nine- or ten-membered cycloalkyl, cycloalkenyl, heterocyclic, aryl, heteroaryl, or a six- to thirty-membered crown ether or heterocrown ether;
R² is selected from a bond, a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic, aryl and heteroaryl;
R³ is selected from a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic, aryi and heteroaryl;
R⁴ is the second portion and is selected from the group consisting of hydrogen, a carboxylate, a thiocarboxylate, an amide, an imide, a hydrazine, a sulfonate, a sulfoxide, a sulfone, a sulfite, a phosphate, a phosphonate, a phosphonium, an alcohol, a thiol, an amine, an ammonium, an alkyl ammonium a nitrate, a sugar moiety, and a five-, six-, seven-, eight-, nine- or ten-membered cycloalkyl, cycloalkenyl, cycloalkynyl, heterocyclic, aryl, or heteroaryl;
*a* is 1, 2, 3 or 4;
*b* is 0, 1, 2 or 3;
*c* is 0, 1, 2 or 3; and
*d* is 0, 1, 2 or 3, wherein when *d* is 2 or 3 the R³ groups can be the same or different or can be linked together to form a five-, six-, seven-, eight-, nine- or ten-membered cycloalkyl, cycloalkenyl, heterocyclic, aryl, or heteroaryl.

15. The water-soluble nanocrystal of claim 14, wherein R' is a thiol, a phosphine, a phosphine oxide, or an amine.

16. The water-soluble nanocrystal of claim 14 ,wherein R² contains between 6 and 20 atoms.

17. The water-soluble nanocrystal of claim 16 ,wherein R² is a linear alkylene, alkenylene, alkynylene, heteroalkylene, heteroalkenylene, heteroalkynylene, a cycloalkyl or a heterocyclic.

18. The water-soluble nanocrystal of claim 14 ,wherein *b* is 1, 2 or 3, and R³ contains between 6 and 20 atoms

19. The water-soluble nanocrystal of claim 18 ,wherein R³ is a linear alkylene, alkenylene, alkynylene, heteroalkylene, heteroalkenylene, heteroalkynylene, a cycloalkyl or a heterocyclic.

20. The water-soluble nanocrystal of claim 14 , wherein R⁴ is a carboxylate (-COO⁻), a phosphonate (-PO₃⁻), a sulfonate (-SO₃⁻) or an ammonium (-N⁺HRR').

21. The water-soluble nanocrystal of claim 1 or 2, wherein the molecule comprises structural formula (V),
(V) ⁅Y²(R¹)⁆_{m'}-R²⁅X²(R⁴)⁆_{n'}.
wherein:
X² and Y² are the same or different and are mer units selected from the group consisting of acrylate, styrene, imide, acrylamide, ethylene, vinyl, diacetylene, phenylene-vinylene, amino acid, sugar, sulfone, pyrrole, imidazole, thiophene and ether;
R¹ is the first portion and is selected from the group consisting of heteroalkyl, heteroalkenyl, heteroalkynyl, -OR, -SR, -NHR, -NR'R", -N(O)HR, -N(O)R'R", -PHR, -PR'R", -P(NR'R")NR'R",-P(O)R'R", -P(O)(NR'R")NR'R", -P(O)(OR')OR", -P(O)OR, -P(O)NR'R", -P(S)(OR')OR", and -P(S)OR, wherein R, R' and R" are independently selected from the group consisting of H, a branched or unbranched alkyl, a branched or unbranched alkenyl, a branched or unbranched alkynyl, a branched or unbranched heteroalkyl, a branched or unbranched heteroalkenyl and a branched or unbranched heteroalkynyl, with the proviso that when a is greater than 1 the R¹ groups can be the same of different or can be linked to form a six-, seven-, eight-, nine- or ten-membered cycloalkyl, cycloalkenyl, heterocyclic, aryl, heteroaryl, or a six- to thirty-membered crown ether or heterocrown¹ether;
R² is selected from a bond, a branched or unbranched alkylene, a branched or unbranched alkenylene, a branched or unbranched heteroalkylene, a branched or unbranched heteroalkenylene, cycloalkyl, cycloalkenyl, cycloalkynyl, heterocyclic, aryl and heteroaryl;
R⁴ is the second portion and is selected from the group consisting of hydrogen, a carboxylate, a thiocarboxylate, an amide, an imide, a hydrazine, a sulfonate, a sulfoxide, a sulfone, a sulfite, a phosphate, a phosphonate, a phosphonium, an alcohol, a thiol, an amine, an ammonium, an alkyl ammonium a nitrate, a sugar moiety, and a five-, six-, seven-, eight-, nine- or ten-membered cycloalkyl, cycloalkenyl, cycloalkynyl, heterocyclic, aryl, or heteroaryl;
m' is in the range of about 3 to 100; and
n' is in the range of about 3 to 100.

22. The water-soluble nanocrystal of claim 21 ,wherein the molecule comprises the formula, 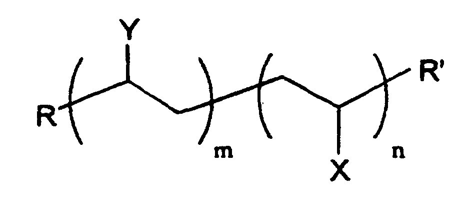 wherein:
X is the same or different and is S, N, P or O=P, can include other substitueuts in order to satisfy valence requirements; Y is a hydrophilic moiety; R is H or a polar moiety; R' is H or a non-polar moiety; *m* is in the range about 3 to 100; and *n* is in the range about 3 to 100.

23. The water-soluble nanocrystal of claim 10 or 12, wherein n is in the range of 10 to 12.

24. The water-soluble nanocrystal of claim 1, 2, 14 or 21 wherein the molecule is a multidentate ligand.

25. The water-soluble nanocrystal of claim 1 or 2, wherein the nanocrystal core is a Group II-VI, Group III-V or Group IV semiconductor.

26. The water-soluble nanocrystal of claim 1, 2 or 25 ,wherein the core comprises CdS, CdSe. CdTe, ZnS, ZnSe, ZnTe, MgTe, GaAs, GaP, GaSb, GaN, HgS, HgSe, HgTe, InAs, InP, InSb, InN, AlAs, AlP, AlSb, AlS, PbS, PbSe, Ge, Si, an alloy thereof, or a mixture thereof

27. The water-soluble nanocrystal of claim 25 or 26 wherein the shell comprises ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AIN, AlP, AlSb, an alloy thereof, or a mixture thereof

28. The water-soluble nanocrystal of claim 1; 2, 25 o r 2 6 wherein the core is CdSe and the shell is ZnS.

29. The water-soluble nanocrystal of claim 1, 2, 25, 2 6 , 2 7 o r 28, wherein the core is a member of a monodisperse particle population.

30. The water-soluble nanocrystal of claim 29, wherein the monodisperse particle population is **characterized in that** when irradiated the population emits light in a spectral range less than about 40 nm full width at half maximum (FWHM), preferably no more than about 25 nm FWHM.

31. The water-soluble nanocrystal of claim 2 9 , wherein the monodisperse particle population is **characterized in that** it exhibits no more than about a 10% rms deviation in the diameter of the core, preferably no more than about 5% rms deviation in the diameter of the core.

32. The water soluble nanocrystal of claim 2 wherein the inner layer comprises a coordinating lyophilic compound

33. The water soluble nanocrystal of claim 32, wherein the coordinating lyophilic compound is selected from the group consisting of trialkyl phosphines, trialkyl phosphine oxides and alkyl amines.

34. The water soluble nanocrystal of claim .2 wherein the outer layer of the bilayer comprises a surfactant.

35. The water soluble nanocrystal of claim 34, wherein the surfactant is selected from the group consisting of sodium dioctyl sulfosuccinate, C₁₂H₂₅(OCH₂CH₂)₂₃OH, C₁₈H₃₇(OCH₂CH₂)₁₀OH and C₁₈H₃₇(OCH₂CH₂)₂₀ OH.

36. The water soluble nanocrystal of claim 1 or 2, wherein the water soluble nanocrystal is dispersed or dissolved in an aqueous medium.

## Patentansprüche

1. Wasserlöslicher Halbleiternanokristall, der zu Energieemission in der Lage ist, mit:
einem Halbleiternanokristallkern mit einer ausgewählten Bandlückenenergie,
einer Hüllenschicht, die den Halbleiternanokristallkern überdeckt, wobei die Hülle ein Halbleitermaterial umfaßt, welches eine Bandlückenenergie größer als jene des Kerns hat,
einer äußeren Schicht, die ein Molekül mit einem ersten Anteil, welcher wenigstens eine Bindungsgruppe für die Befestigung an dem Nanokristall umfaßt, und einem zweiten Anteil mit wenigstens einer hydrophilen Gruppe umfaßt.

2. Wasserlöslicher Halbleiternanokristall nach Anspruch 1, worin die äußere Schicht weiterhin
eine Doppelschicht, die die Hülle überdeckt, ist, wobei die Doppelschicht
eine innere Schicht mit Affinität zu der Hülle und
eine äußere Schicht, die ein Molekül mit einer hydrophilen Gruppe besitzt, die von der inneren Schicht durch einen hydrophoben Bereich angrenzend an die Innenschicht beabstandet ist.

3. Wasserlöslicher Nanokristall nach Anspruch 1, bei dem die Bindungsgruppe einen Rest umfaßt, der aus der Gruppe bestehend aus Aminen, Thiolen, Phosphinen, Phosphinoxiden und Aminoxiden ausgewählt ist.

4. Wasserlöslicher Nanokristall nach Anspruch 1, bei dem die hydrophile Gruppe eine geladene oder polare Gruppe ist.

5. Wasserlöslicher Nanokristall nach Anspruch 1, bei dem die hydrophile Gruppe aus der Gruppe ausgewählt ist, die aus Carbonsäure, Carboxylat (-CO₂⁻), Sulfonat (-SO₃), Hydroxid (-OH), Alkoxiden, Ammoniumsalzen (-NH₄⁺), Phosphat (-PO₄⁻²) und Phosphonat (-PO₃⁻⁷) besteht.

6. Wasserlöslicher Nanokristall nach Anspruch 1, bei dem die hydrophile Gruppe eine ungesättigte hydrophile Gruppe umfaßt, die vernetzbar oder polymerisierbar ist.

7. Wasserlöslicher Nanokristall nach Anspruch 6, bei dem die ungesättigte hydrophile Gruppe aus der Gruppe ausgewählt ist, die aus Methacrylsäure, Acrylsäure und hydrophil derivatisiertem Styrol besteht.

8. Wasserlöslicher Nanokristall nach Anspruch 1, bei dem die Verbindung zwei oder mehr hydrophile Gruppen umfaßt.

9. Wasserlöslicher Nanokristall nach Anspruch 1, worin der erste Anteil von dem zweiten Anteil durch einen hydrophoben Bereich beabstandet ist.

10. Wasserlöslicher Nanokristall nach Anspruch 9, bei dem der hydrophobe Bereich eine Kohlenwasserstoffkette der Formel -(CH₂)ₙ-, worin n größer als oder gleich sechs ist.

11. Wasserlöslicher Nanokristall nach Anspruch 1 oder 2, bei dem das Molekül die Strukturformel (I)
(I) H₂X((CH₂)ₙCO₂H)_{y}
oder eines Salzes derselben umfaßt, worin
X der erste Anteil des Liganden ist und S, N, P oder O=P bedeutet,
n größer als oder gleich 6 ist und
x und y so ausgewählt sind, daß sie die Wertigkeitserfordernisse von X befriedigen.

12. Wasserlöslicher Nanokristall nach Anspruch 1 oder 2, bei dem das Molekül die Strukturformel (II) hat 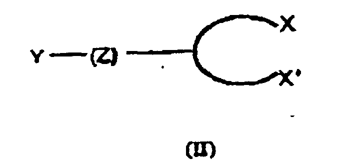 worin:
Y der hydrophile Rest ist,
Z ein hydrophober Bereich mit einem Grundgerüst von wenigstens sechs Atomen ist,
X und X' einzeln oder zusammengenommen die Bindungsgruppen sind, gleich oder verschieden sind und aus der Gruppe S, N, P und O=P ausgewählt sind oder aneinander gebunden sind, um einen 5-gliedrigen bis 8-gliedrigen Ring bei Koordination an der Nanokristallfäche zu bilden.

13. Wasserlöslicher Nanokristall nach Anspruch 1 oder 2, bei dem das Molekül die Strukturformel (III) hat 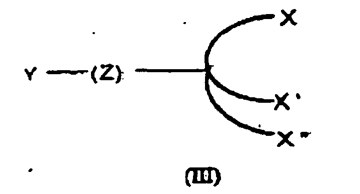 worin:
Y der hydrophile Rest ist,
Z ein hydrophober Bereich mit einem Grundgerüst von wenigstens sechs Atomen ist,
X, X' und X" einzeln oder zusammengenommen Bindungsgruppen sind, gleich oder verschieden sind und aus der Gruppe S, N, P und O=P ausgewählt sind oder aneinander gebunden sind, um einen 5-gliedrigen bis 8-gliedrigen Ring bei Koordination an der Nanokristallfläche zu bilden.

14. Wasserlöslicher Nanokristall nach Anspruch 1 oder 2, bei dem das Molekül die Strukturformel (IV) hat
(IV) (R¹)ₐ-R²-[(R³)_{b}(R⁴)_{c}]_{d}
worin:
R¹ der erste Abschnitt ist und aus der Gruppe ausgewählt ist, die aus Heteroalkyl, Heteroalkenyl, Heteroalkinyl, -OR, -SR, -NHR,- NR'R", -N(O)HR, -N(O)R'R", -PHR, -PR'R", -P(NR'R")NR'R", -P(O)R'R", -P(O)(NR'R")NR'R", -P(O)(OR')OR", -P(O)OR, -P(O)NR'R", -P(S)(OR')OR" und -P(S)OR besteht, worin R, R' und R" unabhängig voneinander aus der Gruppe ausgewählt sind, die aus H, aus einem verzweigten oder unverzweigten Alkyl, einem verzweigten oder unverzweigten Alkenyl, einem verzweigten oder unverzweigten Alkinyl, einem verzweigten oder unverzweigten Heteroalkyl, einem verzweigten oder unverzweigten Heteroalkenyl und einem verzweigten oder unverzweigten Heteroalkinyl, besteht, wobei, wenn a größer als 1 ist, die Gruppen R' gleich oder verschieden sein können oder unter Bildung von sechs-, sieben-, acht-, neun- oder zehn-gliedrigem Cycloalkyl, Cycloalkenyl, heterocyclischem Aryl, Heteroaryl oder eines sechs- bis dreißig-gliedrigen Kronenethers oder Heterokronenethers verbunden sein können, R² aus einer Bindung, einem verzweigten oder unverzweigten Alkylen, einem verzweigten oder unverzweigten Alkenylen, einem verzweigten oder unverzweigten Heteroalkylen, einem verzweigten oder unverzweigten Heteroalkenylen, Cycloalkyl, Cycloalkenyl, Cycloalkinyl, heterocyclischem Aryl und Heteroaryl ausgewählt ist,
R^{3'} aus einem verzweigten oder unverzweigten Alkylen, einem verzweigten oder unverzweigten Alkenylen, einem verzweigten oder unverzweigten Heteroalkylen, einem verzweigten oder unverzweigten Heteroalkenylen, Cycloalkyl, Cycloalkenyl, Cycloalkinyl, heterocyclischem Aryl und Heteroaryl, ausgewählt ist,
R⁴ der zweite Abschnitt ist und aus der Gruppe ausgewählt ist, welche aus Wasserstoff, einem Carboxylat, einem Thiocarboxylat, einem Amid, einem Imid, einem Hydrazid, einem Sulfonat, einem Sulfoxid, einem Sulfon, einem Sulfit, einem Phosphat, einem Phosphonat, einem Phosphonium, einem Alkohol, einem Thiol, einem Amin, einem Ammonium, einem Alkylammonium, einem Nitrat, einem Zuckerrest und einem fünf-, sechs-, sieben-, acht-, neun- oder zehngliedrigen Cycloalkyl, Cycloalkenyl, Cycloalkinyl, heterocyclischem Aryl oder Heteroaryl besteht, wobei
a 1, 2, 3 oder 4 ist,
b 0, 1, 2 oder 3 ist
c 0, 1, 2 oder 3 ist und
d 0, 1, 2 oder 3 ist, worin, wenn d 2 oder 3 ist, die Gruppen R³ gleich oder verschieden sein können oder miteinander zur Bildung von fünf-, sechs-, sieben.-, acht-, neunoder zehngliedrigem Cycloalkyl, Cycloalkenyl, heterocyclischem Aryl oder Heteroaryl verbunden sein können.

15. Wasserlöslicher Nanokristall nach Anspruch 14, worin R' ein Thiol, ein Phosphin, ein Phosphinoxid oder ein Amin ist.

16. Wasserlöslicher Nanokristall nach Anspruch 14, worin R² 6 bis 20 Atome enthält.

17. Wasserlöslicher Nanokristall nach Anspruch 16, worin R² lineares Alkylen, Alkenylen, Alkinylen, Heteroalkylen, Heteroalkenylen, Heteroalkinylen, ein Cycloalkyl oder ein Heterocyclus sein kann.

18. Wasserlöslicher Nanokristall nach Anspruch 14, worin b 1, 2 oder 3 ist und R³ zwischen 6 und 20 Atome enthält.

19. Wasserlöslicher Nanokristall nach Anspruch 18, worin R³ ein lineares Alkylen, Alkenylen, Alkinylen, Heteroalkylen, Heteroalkenylen, Heteroalkinylen, Cycloalkyl oder ein Heterocyclus ist.

20. Wasserlöslicher Nanokristall nach Anspruch 14, worin R⁴ ein Carboxylat (-COO⁻), ein Phosphonat (-PO₃), Sulfonat (-SO₃) oder ein Ammonium (-N³HRR⁴) ist.

21. Wasserlöslicher Nanokristall nach Anspruch 1 oder 2, bei dem das Molekül die Strukturformel (V) besitzt
(V) [*-Y*²(*R*')-]_{*m*}-*R*²[*-X*²(*R*⁴)-]_{*n*}
worin
X² und Y² gleich oder verschieden sind und mer-Einheiten sind, die aus der Gruppe ausgewählt sind, die aus Acrylat, Styrol, Imid, Acrylamid, Ethylen, Vinyl, Diacetylen, Phenylen-Vinylen, Aminosäure, Zucker, Sulfon, Pyrrol, Imidazol, Thiophen und Ether besteht, R' der erste Abschnitt ist und aus der Gruppe ausgewählt wird, die aus Heteroalkyl, Heteroalkenyl, Heteroalkinyl, -OR, -SR, -NHR, -NR'R", -N(O)HR, -N(O)R'R", -PHR, -PR'R", -P(NR'R")NR'R", -P(O)R'R", -P(O)CNR'R")NR'R", -P(O)(OR')OR", -P(O)OR, -P(O)NR'R", -P(S)(OR')OR" und -P(S)OR besteht, worin R, R' und R" unabhängig voneinander aus der Gruppe ausgewählt sind, die aus H, einem verzweigten oder unverzweigten Alkyl, einem verzweigten oder unverzweigten Alkenyl, einem verzweigten oder unverzweigten Alkinyl, einem verzweigten oder unverzweigten Heteroalkyl, einem verzweigten oder unverzweigten Heteroalkenyl und einem verzweigten oder unverzweigten Heteroalkinyl besteht, wobei, wenn a größer als 1 ist, die Gruppen R' gleich oder verschieden sein können, oder ein sechs-, sieben-, acht-, neun- oder zehngliedriges Cycloalkyl, Cycloalkenyl, heterocyclisches Aryl, Heteroaryl, oder ein sechs- bis dreißiggliedriger Kronenther oder Heterokronenether sein können,
R² aus einer Bindung, einem verzweigten oder unverzweigten Alkylen, einem verzweigten oder unverzweigten Alkenylen, einem verzweigten oder unverzweigten Heteroalkylen, einem verzweigten oder unverzweigten Heteroalkenylen, einem Cycloalkyl, Cycloalkenyl, Cycloalkinyl, heterocyclischen Aryl und Heteroaryl besteht,
R⁴ der zweite Abschnitt ist und aus der Gruppe ausgewählt ist, welche aus Wasserstoff, einem Carboxylat, Thiocarboxylat, Amid, Imid, Hydrazin, Sulfonat, Sulfoxid, Sulfon, Sulfit, Phosphat, Phosphonat, Phosphonium, einem Alkohol, einem Thiol, einem Amin, einem Ammonium, einem Alkylammonium, einem Nitrat, einem Zuckerrest und einem fünf-, sechs-, sieben-, acht-, neun- oder zehngliedrigen Cycloalkyl, Cycloalkenyl, Cycloalkinyl, herterocyclischem Aryl oder Heteroaryl besteht,
m' im Bereich von etwa 3 bis 100 liegt und
n' in dem Bereich von etwa 3 bis 100 liegt.

22. Wasserlöslicher Nanokristall nach Anspruch 21, worin das Molekül die Formel 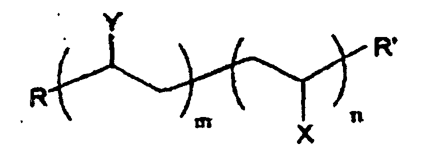 umfaßt, worin
X gleich oder verschieden ist und S, N, P oder O=P ist, andere Substituenten einschließen kann, um Wertigkeitserfordernissen zu genügen, Y ein hydrophiler Rest ist, R H oder ein polarer Rest ist, R' H oder ein nicht-polarer Rest ist, m im Bereich von etwa 3 bis 100 liegt und n im Bereich von etwa 3 bis 100 liegt.

23. Wasserlöslicher Nanokristall nach Anspruch 10 oder 12, worin n im Bereich von 10 bis 12 liegt.

24. Wasserlöslicher Nanokristall nach Anspruch 1, 2, 14 oder 21, worin das Molekül ein Multidentatligand ist.

25. Wasserlöslicher Nanokristall nach Anspruch 1 oder 2, worin der Nanokristallkern ein Halbleiter der Gruppe II-VI, der Gruppe III-V oder der Gruppe IV ist.

26. Wasserlöslicher Nanokristall nach Anspruch 1, 2 oder 25, worin der Kern CdS, CdSe, CdTe, ZnS, ZnSe, ZnTe, MgTe, GaAs, GaP, GaSb, GaN, HgS, HgSe, HgTe, InAs, InP, InSb, InN, AlAs, AlP, AlSb, AlS, PbS, PbSe, Ge, Si, eine Legierung hiervon oder ein Gemisch hiervon umfaßt.

27. Wasserlöslicher Nanokristall nach Anspruch 25 oder 26, bei dem die Hülle ZnO, ZnS, ZnSe, ZnTe, CdO, CdS, CdSe, CdTe, MgS, MgSe, GaAs, GaN, GaP, GaAs, GaSb, HgO, HgS, HgSe,HgTe, InAs, InN, InP, InSb, AlAs, AIN, AlP, AlSb, eine Legierung hiervon oder ein Gemisch hiervon umfaßt.

28. Wasserlöslicher Nanokristall nach Anspruch 1, 2, 25 oder 26, worin der Kern CdSe und die Hülle ZnS ist.

29. Wasserlöslicher Nanokristall nach Anspruch 1, 2, 25, 26, 27 oder 28, bei dem der Kern ein Glied einer monodispersen Teilchenpopulation ist.

30. Wasserlöslicher Nanokristall nach Anspruch 29, bei dem die monodisperse Teilchenpopulation **dadurch gekennzeichnet ist, daß** sie, wenn sie bestrahlt wird, Licht in einem Spektralbereich von weniger als etwa 40 nm, volle Breite bei halbem Maximum (FWHM), vorzugsweise nicht mehr als etwa 25 nm FWHM emittiert.

31. Wasserlöslicher Nanokristall nach Anspruch 29, bei dem die monodisperse Teilchenpopulation **dadurch gekennzeichnet ist, daß** sie nicht mehr als etwa 10% Umdrehungen je Minute Abweichung im Durchmesser des Kerns, vorzugsweise nicht mehr als etwa 5% Umdrehungen je Minute Abweichung im Durchmesser des Kerns zeigt.

32. Wasserlöslicher Nanokristall nach Anspruch 2, bei dem die innere Schicht eine koordinierende lyophile Verbindung umfaßt.

33. Wasserlöslicher Nanokristall nach Anspruch 32, bei dem die koordinierende lyophile Verbindung aus der Gruppe ausgewählt ist, die aus Trialkylphosphinen, Trialkylphosphinoxiden und Alkylaminen besteht.

34. Wasserlöslicher Nanokristall nach Anspruch 2, bei dem die Außenschicht der doppelschichtigen Struktur ein oberflächenaktives Mittel umfaßt.

35. Wasserlöslicher Nanokristall nach Anspruch 34, bei dem das oberflächenaktive Mittel aus der Gruppe ausgewählt ist, die aus Natriumdioctylsulfosuccinat, C₁₂H₂₅(OCH₂CH₂)₂₃OH, C₁₈H₃₇(OCH₂CH₂)₁₀OH und C₁₈H₃₇(OCH₂CH₂)₂₀OH besteht.

36. Wasserlöslicher Nanokristall nach Anspruch 1 oder 2, bei dem der wasserlösliche Nanokristall in einem wäßrigen Medium dispergiert oder gelöst ist.

## Revendications

1. Nanocristal semiconducteur hydrosoluble apte à la transmission d'énergie, comprenant :
un noyau de nanocristal semiconducteur ayant une énergie choisie de bande interdite ;
une couche d'enveloppe recouvrant le noyau de nanocristal semiconducteur, l'enveloppe étant constituée d'une matière semiconductrice ayant une énergie de bande interdite supérieure à celle du noyau ;
une couche extérieure comprenant une molécule comportant une première partie comprenant au moins un groupe de liaison pour la fixation au nanocristal et une seconde partie comprenant au moins un groupe hydrophile.

2. Nanocristal semiconducteur hydrosoluble suivant la revendication 1, dans lequel la couche extérieure comprend en outre
une double couche recouvrant l'enveloppe, la double couche comprenant :
une couche intérieure présentant une affinité pour l'enveloppe ; et
une couche extérieure comprenant une molécule comportant un groupe hydrophile espacé de la couche intérieure par une région hydrophobe adjacente à la couche intérieure.

3. Nanocristal hydrosoluble suivant la revendication 1, dans lequel le groupe de liaison comprend un groupement choisi dans le groupe consistant en des amines, des thiols, des phosphines, des oxydes de phosphines et des oxydes d'amines.

4. Nanocristal hydrosoluble suivant la revendication 1, dans lequel le groupe hydrophile est un groupe chargé ou polaire.

5. Nanocristal hydrosoluble suivant la revendication 1, dans lequel le groupe hydrophile est choisi dans le groupe consistant en des groupes acide carboxylique, carboxylate (-CO₂⁻), sulfonate (-SO₃), hydroxyde (-OH), alcoolate, sel d'ammonium (-NH₄⁺) et phosphate (-PO₄⁻²) et phosphonate (-PO₃⁻²).

6. Nanocristal hydrosoluble suivant la revendication 1, dans lequel le groupe hydrophile comprend un groupe hydrophile insaturé qui est réticulable ou polymérisable.

7. Nanocristal hydrosoluble suivant la revendication 6, dans lequel le groupe hydrophile insaturé est choisi dans le groupe consistant en l'acide méthacrylique, l'acide acrylique et le styrène transformé en dérivé hydrophile.

8. Nanocristal hydrosoluble suivant la revendication 1, dans lequel le composé comprend deux ou plus de deux groupes hydrophiles.

9. Nanocristal hydrosoluble suivant la revendication 1, dans lequel la première partie est espacée de la seconde partie par une région hydrophobe.

10. Nanocristal hydrosoluble suivant la revendication 9, dans lequel la région hydrophobe comprend une chaîne hydrocarbonée de formule -(CH₂)ₙ-, dans laquelle n est supérieur ou égal à 6.

11. Nanocristal hydrosoluble suivant la revendication 1 ou 2, dans lequel la molécule répond à la formule structurale (I),
(I) H₂X ((CH₂)ₙCO₂H)_{y}
ou est un de ses sels, formule dans laquelle :
X représente la première partie du ligand et représente S, N, P ou O=P ;
n est supérieur ou égal à 6 ; et
z et y sont choisis de manière à satisfaire les besoins de valence de X.

12. Nanocristal hydrosoluble suivant la revendication 1 ou 2, dans lequel la molécule répond à la formule structurale (II) dans laquelle, 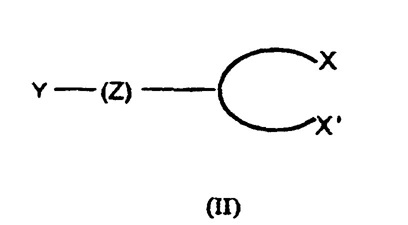 dans laquelle :
Y représente un groupement hydrophile ;
Z représente une région hydrophobe ayant un squelette d'au moins six atomes ;
X et X' représentent, individuellement ou conjointement, les groupes de liaison, sont identiques ou différents et sont choisis dans le groupe consistant en S, N, P et O=P, ou bien sont liés l'un à l'autre pour former un noyau pentagonal à octogonal par coordination à la surface du nanocristal.

13. Nanocristal hydrosoluble suivant la revendication 1 ou 2, dans lequel la molécule répond à la formule structurale (III), 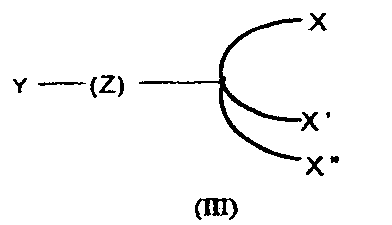 dans laquelle :
Y représente le groupement hydrophile ;
Z représente une région hydrophobe ayant un squelette d'au moins six atomes ;
X, X' et X" représentent, individuellement ou conjointement, des groupes de liaison, sont identiques ou différents et sont choisis dans le groupe consistant en S, N, P et O=P, ou sont liés les uns aux autres pour former un noyau pentagonal à octogonal par coordination à la surface du nanocristal.

14. Nanocristal hydrosoluble suivant la revendication 1 ou 2, dans lequel la molécule répond à la formule structurale (IV),
(IV) (R¹)ₐ-R²-[(R³)_{b}(R⁴)_{c}]_{d}
dans laquelle :
R¹ représente la première partie et est choisi dans le groupe consistant en des groupes hétéroalkyle, hétéroalcényle, hétéroalcynyle, -OR, -SR, -NHR, -NR'R", -N(O)HR, -N(O)R'R", -PHR, -PR'R", -P(NR'R")NR'R", -P(O)R'R", -P(O) (NR'R")NR'R", -P(O) (OR')OR", -P(O)OR, -P(O)NR'R", -P(S)(OR')OR" et -P(S)OR, dans lesquels R, R' et R" sont choisis indépendamment dans le groupe consistant en H, un groupe alkyle ramifié ou non ramifié, un groupe alcényle ramifié ou non ramifié, un groupe alcynyle ramifié ou non ramifié, un groupe hétéroalkyle ramifié ou non ramifié, un groupe hétéroalcényle ramifié ou non ramifié et un groupe hétéroalcynyle ramifié ou non ramifié, sous réserve que, lorsque a est supérieur à 1, les groupes R¹ puissent être identiques ou différents ou puissent être liés pour former un groupe cycloalkyle, cycloalcényle, hétérocyclique, aryle ou hétéroaryle hexa-, hepta-, octo-, nona- ou décagonal ou un éther en couronne ou hétéroéther en couronne de six à trente membres ;
R² est choisi entre une liaison, un groupe alkylène ramifié ou non ramifié, un groupe alcénylène ramifié ou non ramifié, un groupe hétéroalkylène ramifié ou non ramifié, un groupe hétéroalcénylène ramifié ou non ramifié, des groupes cycloalkyle, cycloalcényle, cycloalcynyle, hétérocycliques, aryle et hétéroaryle ;
R³ est choisi entre un groupe alkylène ramifié ou non ramifié, un groupe alcénylène ramifié ou non ramifié, un groupe hétéroalkylène ramifié ou non ramifié, un groupe hétéroalcénylène ramifié ou non ramifié, des groupes cycloalkyle, cycloalcényle, cycloalcynyle, hétérocycliques, aryle et hétéroaryle ;
R⁴ représente la seconde partie et est choisi dans le groupe consistant en un atome d'hydrogène, un carboxylate, un thiocarboxylate, un amide, un imide, une hydrazine, un sulfonate, un sulfoxyde, une sulfone, une sulfite, un phosphate, un phosphonate, un phosphonium, un alcool, un thiol, une amine, un ammonium, un alkylammonium, un nitrate, un groupement sucre et un groupe cycloalkyle, cycloalcényle, cycloalcynyle, hétérocyclique, aryle ou hétéroaryle penta-, hexa-, hepta-, octo-, nona- ou ) décagonal ;
a est égal à 1, 2, 3 ou 4 ;
b est égal à 0, 1, 2 ou 3 ;
c est égal à 0, 1, 2 ou 3 ; et
d est égal à 0, 1, 2 ou 3, où, lorsque d est égal à 2 ou 3, les groupes R³ peuvent être identiques ou différents et peuvent être liés les uns aux autres pour former un groupe cycloalkyle, cycloalcényle, hétérocyclique, aryle ou hétéroaryle penta, hexa-, hepta-, octo-, nona- ou décagonal.

15. Nanocristal hydrosoluble suivant la revendication 14, dans lequel R¹ représente un thiol, une phosphine, un oxyde de phosphine ou une amine.

16. Nanocristal hydrosoluble suivant la revendication 14, dans lequel R² contient 6 à 20 atomes.

17. Nanocristal hydrosoluble suivant la revendication 16, dans lequel R² représente un groupe alkylène, alcénylène, alcynylène, hétéroalkylène, hétéroalcénylène ou hétéroalcynylène linéaire, un groupe cycloalkyle ou un groupe hétérocyclique.

18. Nanocristal hydrosoluble suivant la revendication 14, dans lequel b est égal à 1, 2 ou 3 et R³ contient 6 à 20 atomes.

19. Nanocristal hydrosoluble suivant la revendication 18, dans lequel R¹ représente un groupe alkylène, alcénylène, alcylylène, hétéroalkylène, hétéroalcénylène ou hétéroalcynylène linéaire, un groupe cycloalkyle ou un groupe hétérocyclique.

20. Nanocristal hydrosoluble suivant la revendication 14, dans lequel R⁴ représente un carboxylate (-COO⁻), un phosphonate (-PO₃⁻), un sulfonate (-SO₃⁻) ou un ammonium (-N⁺HRR').

21. Nanocristal hydrosoluble suivant la revendication 1 ou 2, dans lequel la molécule comprend la formule structurale (V),
(V) ⁅Y²(R¹)⁆_{m'}-R²⁅X²(R⁴)⁆ₙ'
dans laquelle :
X² et Y² sont identiques ou différents et représentent des motifs monomères choisis dans le groupe consistant en acrylate, styrène, imide, acrylamide, éthylène, vinyle, diacétylène, phénylène-vinylène, aminoacide, sucre, sulfone, pyrrole, imidazole, thiophène et éther ;
R¹ représente la première partie et est choisi dans le groupe consistant en des groupes hétéroalkyle, hétéroalcényle, hétéroalcynyle, -OR, -SR, -NHR, -NR'R", -N(O)HR, -N(O)R'R", -PHR, -PR'R", -P(NR'R")NR'R", -P(O)R'R", - P(O)(NR'R")NR'R", -P(O)(OR')OR", -P(O)OR, -P(O)NR'R", -P(S)(OR')OR" et -P(S)OR, dans lesquels R, R' et R" sont choisis indépendamment dans le groupe consistant en H, un groupe alkyle ramifié ou non ramifié, un groupe alcényle ramifié ou non ramifié, un groupe alcynyle ramifié ou non ramifié, un groupe hétéroalkyle ramifié ou non ramifié, un groupe hétéroalcényle ramifié ou non ramifié et un groupe hétéroalcynyle ramifié ou non ramifié, sous réserve que, lorsque a est supérieur à 1, les groupe R¹ puissent être identiques ou différents ou puissent être liés pour former un groupe cycloalkyle, cycloalcényle, hétérocyclique, aryle ou hétéroaryle hexa-, hepta-, octo-, nona- ou décagonal ou un éther en couronne ou hétéroéther en couronne de six à trente membres ;
R² est choisi entre une liaison, un groupe alkylène ramifié ou non ramifié, un groupe alcénylène ramifié ou non ramifié, un groupe hétéroalkylène ramifié ou non ramifié, un groupe hétéroalcénylène ramifié ou non ramifié, des groupes cycloalkyle, cycloalcényle, cycloalcynyle, hétérocycliques, aryle et hétéroaryle ;
R⁴ représente la seconde partie et est choisi dans le groupe consistant en un atome d'hydrogène, un carboxylate, un thiocarboxylate, un amide, un imide, une hydrazine, un sulfonate, un sulfoxyde, une sulfone, une sulfite, un phosphate, un phosphonate, un phosphonium, un alcool, un thiol, une amine, un ammonium, un alkylammonium, un nitrate, un groupement sucre et un groupe cycloalkyle, cycloalcényle, cycloalcynyle, hétérocyclique, aryle ou hétéroaryle penta-, hexa-, hepta-, octo-, nona- ou décagonal ;
m' est compris dans l'intervalle d'environ 3 à 100 ; et
n' est compris dans l'intervalle d'environ 3 à 100.

22. Nanocristal hydrosoluble suivant la revendication 21, dans lequel la molécule comprend la formule, 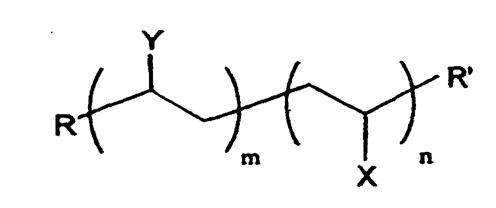 dans laquelle :
les groupes X sont identiques ou différents et représentent S, N, P et O=P, et peuvent comprendre d'autres substituants afin de satisfaire les besoins de valence ; Y représente un groupement hydrophile ; R représente H ou un groupement polaire ; R' représente H ou un groupement non polaire ; m est compris dans l'intervalle d'environ 3 à 100 et n est compris dans l'intervalle d'environ 3 à 100.

23. Nanocristal hydrosoluble suivant la revendication 10 ou 12, dans lequel n est compris dans l'intervalle de 10 à 12.

24. Nanocristal hydrosoluble suivant la revendication 1, 2, 14 ou 21, dans lequel la molécule est un ligand multidenté.

25. Nanocristal hydrosoluble suivant la revendication 1 ou 2, dans lequel le noyau du nanocristal est un semiconducteur des Groupes II-VI, des Groupes III-V ou du Groupe IV.

26. Nanocristal hydrosoluble suivant la revendication 1, 2 ou 25, dans lequel le noyau comprend CdS, CdSe, CdTe, ZnS, ZnSe, ZnTe, MgTe, GaAs, GaP, GaSb, GaN, HgS, HgSe, HgTe, InAs, InP, InSb, InN, AlAs, AlP, AlSb, AlS, PbS, PbSe, Ge, Si, un de leurs alliages ou un de leurs mélanges.

27. Nanocristal hydrosoluble suivant la revendication 25 ou 26, dans lequel l'enveloppe comprend ZnO, ZnS, ZnSe, ZnTe, CdO, CdS, CdSe, CdTe, MgS, MgSe, GaAs, GaN, GaP, GaAs, GaSb, HgO, HgS, HgSe, HgTe, InAs, InN, InP, InSb, AlAs, AlN, AlP, AlSb, un de leurs alliages ou un de leurs mélanges.

28. Nanocristal hydrosoluble suivant la revendication 1, 2, 25 ou 26, dans lequel le noyau est constitué de CdSe et l'enveloppe est constituée de ZnS.

29. Nanocristal hydrosoluble suivant la revendication 1, 2, 25, 26, 27 ou 28, dans lequel le noyau est un membre d'une population de particules monodispersées.

30. Nanocristal hydrosoluble suivant la revendication 29, dans lequel la population de particules monodispersées est **caractérisée en ce que**, lors de l'irradiation, la population émet de la lumière sur une plage spectrale inférieure à environ 40 nm de largeur à mi-hauteur (FWHM), de préférence non supérieure à environ 25 nm FWHM.

31. Nanocristal hydrosoluble suivant la revendication 29, dans lequel la population de particules monodispersées est **caractérisée en ce qu'**elle présente un écart non supérieur à environ 10% rms du diamètre du noyau, de préférence un écart non supérieur à environ 5% rms du diamètre du noyau.

32. Nanocristal hydrosoluble suivant la revendication 2, dans lequel la couche intérieure comprend un composé lyophile coordinant.

33. Nanocristal hydrosoluble suivant la revendication 32, dans lequel le composé lyophile coordinant est choisi dans le groupe consistant en des trialkylphosphines, des oxydes de trialkylphosphines et des alkylamines.

34. Nanocristal hydrosoluble suivant la revendication 2, dans lequel la couche extérieure de la double couche comprend un agent tensioactif.

35. Nanocristal hydrosoluble suivant la revendication 34, dans lequel l'agent tensioactif est choisi dans le groupe consistant en dioctylsulfosuccinate de sodium, C₁₂H₂₅(OCH₂CH₂)₂₃OH, C₁₈H₃₇(OCH₂CH₂)₁₀OH et C₁₈H₃₇(OCH₂CH₂)₂₀OH.

36. Nanocristal hydrosoluble suivant la revendication 1 ou 2, ledit nanocristal hydrosoluble étant dispersé ou dissous dans un milieu aqueux.
